**Europäisches Patentamt**

**European Patent Office**

**Office européen des brevets**

(19)

(11) **EP 0 484 787 B1**

(12) **EUROPÄISCHE PATENTSCHRIFT**

(45) Veröffentlichungstag und Bekanntmachung des Hinweises auf die Patenterteilung:
**10.10.2001 Patentblatt 2001/41**

(51) Int Cl.⁷: **C07K 7/08**, C07K 14/18, A61K 39/12, C12N 15/51, G01N 33/576

(21) Anmeldenummer: **91118349.9**

(22) Anmeldetag: **28.10.1991**

(54) **HCV-spezifische Peptide, Mittel dazu und ihre Verwendung**

HCV-specific peptides, process for obtaining them and their use

Peptides spécifiques pour HCV, procédé pour les synthétiser et leur emploi

(84) Benannte Vertragsstaaten:
**AT BE CH DE DK ES FR GB IT LI LU NL SE**

(30) Priorität: **03.11.1990 DE 4034982**
　　　　　　**19.04.1991 DE 4112743**
　　　　　　**19.06.1991 DE 4120281**
　　　　　　**28.06.1991 DE 4121431**

(43) Veröffentlichungstag der Anmeldung:
**13.05.1992 Patentblatt 1992/20**

(60) Teilanmeldung:
**96112414.6 / 0 770 679**

(73) Patentinhaber: **Dade Behring Marburg GmbH**
**35001 Marburg (DE)**

(72) Erfinder:
* **Krupka, Udo, Dr.**
**W-3550 Marburg (DE)**
* **Stüber, Werner, Dr.**
**W-3551 Lahntal (DE)**
* **Gerken, Manfred, Dr.**
**W-3550 Marburg (DE)**
* **Brust, Stefan, Dr.**
**W-3550 Marburg (DE)**

(56) Entgegenhaltungen:
**EP-A- 0 318 216**　　　**EP-A- 0 363 025**
**EP-A- 0 388 232**　　　**EP-A- 0 419 182**
**EP-A- 0 442 394**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft Polypeptide zur immunchemischen Bestimmung von HCV-spezifischen Antikörpern und HCV-Antigenen sowie für diese Verfahren geeignete Mittel und ihre Verwendung.

Die Erfindung betrifft auch ein immunchemisches Verfahren zum gleichzeitigen Nachweis und/oder zur gleichzeitigen Bestimmung von mehreren verschiedenen Antikörperspezifitäten gegen jeweils unterschiedliche Pathogene in einem einzigen Test.

Die Non A/Non B-Hepatitis (NANBH) wird als übertragbare Krankheit bzw. Gruppe von Krankheitsbildern als virus-assoziiert angesehen und ist von anderen virus-induzierten Krankheitsbildern abgrenzbar, die durch differente Hepatitis-Viren ausgelöst wird, wie Hepatitis A Virus (HAV), Hepatitis B Virus (HBV), Hepatitis D Virus (HDV) und Hepatitis E (HEV). Schließlich lassen sich auch Hepatitiden diagnostizieren, die durch Cytomegalie Virus (CMV) oder Epstein Barr Virus (EBV) hervorgerufen werden.

Auf der Basis epidemiologischer Erhebungen lassen sich entsprechend dem Übertragungsweg mindestens zwei Non A/Non B-Hepatitiden (NANBH) definieren: das epidemische Hepatitis Virus (enterically transmitted NANBV), welches durch Wasser und Nahrungsmittel übertragen wird, sowie das Posttransfusions-Hepatitis Virus (blood transmitted NANBV), das durch Blut, Nadelstiche oder ähnliche Wege übertragen wird. Neben diesen Infektionswegen sind auch Übertragungen bekannt, die als sporadisch auftrende NANBV ("community acquired NANBV") keine offensichtliche Zugehörigkeit zu den beiden genannten Typen aufweisen. Obwohl die genaue Zahl von Agentien bzw. Viren, die eine NANBH auslösen, nicht bekannt ist, wurde das sogenannte Hepatitis C Virus (HCV) als ein ursächlicher Erreger dieser Krankheit kürzlich identifiziert (WO 89/04 669).

Eine klinische Diagnose stützte sich bis vor kurzem hauptsächlich auf die serologische Bestimmung von Antigenen und/oder dagegen gerichtete Antikörper, wobei die Tests spezifisch sind für Parameter aus der Gruppe der Hepatitis-Erreger HAV, HBV, HDV, HEV, CMV oder EBV. Bei diesen sogenannten Ausschlußverfahren wurde nur dann eine NANBH diagnostiziert, wenn die oben genannten Bestimmungen insgesamt negativ waren.

Daneben wurden auch sogenannte Surrogat-Marker verwendet, wie z. B. GPT (Glutamat-Pyruvat-Transaminase, auch als ALT bezeichnet - Alanin Aminotransferase) oder Anti HBc (Hepatitis B core-spezifische Antikörper). Diese Hilfsmittel sind jedoch weder sensitiv noch spezifisch genug, um als zuverlässig gelten zu können. Bei der Untersuchung von Blutspendern kann daher auch nur ein kleiner Teil der Post-Transfusions-Hepatitiden, die bei ca. 10 % der transfundierten Patienten auftritt, vermieden werden. Die Dringlichkeit der Einführung eines spezifischen Tests wird durch die Tatsache unterstrichen, daß für etwa 90 % der Post-Transfusions-Hepatitiden das NANBV verantwortlich gemacht wird. Das Hauptproblem dieser Erkrankung besteht in der Tatsache, daß zwischen 25 und 55 % der Infizierten chronische Leberschäden erleiden.

Mit der Entdeckung des HCV wurde die Basis für einen spezifischen Nachweis von HCV bzw. HCV-spezifischen Antikörpern geschaffen. Die Isolierung und Charakterisierung des HCV bzw. entsprechender cDNA-Replikate von Teilen des HCV-Genoms ist Gegenstand der WO 89/04 669, worin die Einordnung des HCV in die Familie der flavi-ähnlichen Viren vorgenommen wird. Dort wird auch die Verwendung von HCV-Antigen für den Nachweis von HCV-spezifischen Antikörpern beschrieben sowie die Erzeugung von Antikörpern für die diagnostische Bestimmung von Antigen im Patientenblut. Zur Anwendung kommen allerdings gentechnologisch hergestellte Proteine, insbesondere sog. Nicht-Struktur-Proteine (NSP) aus der "Open reading frame region" (ORF), die als Reaktionspartner in immunchemischen Nachweisverfahren Verwendung finden.

Unter einem immunchemischen Nachweis werden im Sinne dieser Erfindung alle Verfahren zusammengefaßt, die als homogene (in Lösung) oder heterogene (mit fester Phase) in vitro Methoden die Bestimmung von Antigenen und/oder Antikörpern der Immunglobulinklassen A, D, E, G oder M (IgA, IgD, IgE, IgG oder IgM) in Körperflüssigkeiten wie Serum, Plasma, Speichel, Liquor oder Urin erlauben. Beispiele für diese auch Immunoassay genannten Verfahren sind Enzymimmunoassay (ELISA oder EIA), Radioimmunoassay (RIA), Immunfluoreszenzassay (IFA), Radioimmunopräzipitation (RIPA), Agar Gel-Diffusion usw.

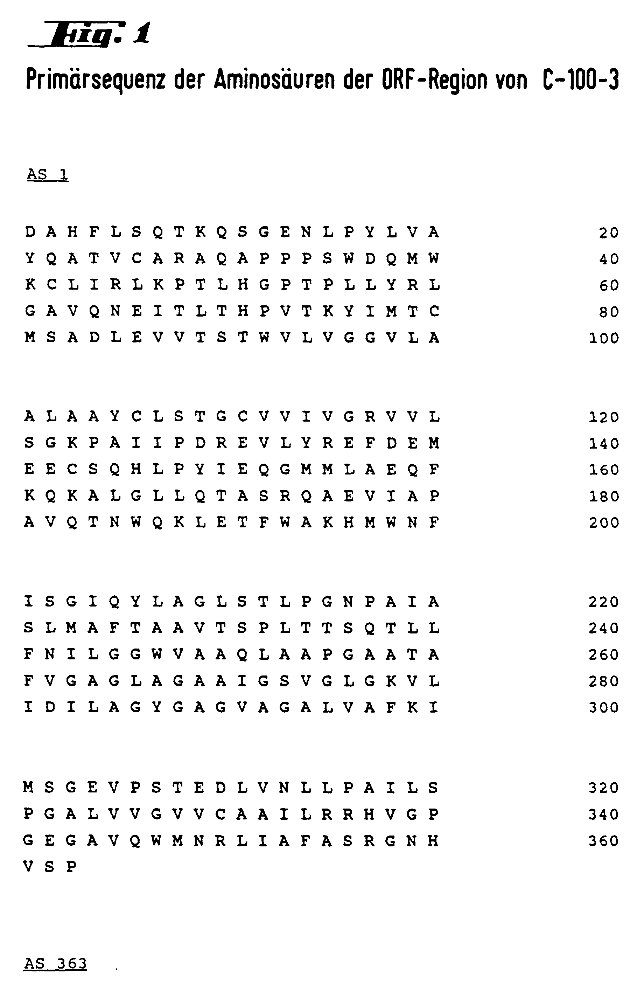

In der WO 89/04 669 wurde beispielsweise zum Nachweis von HCV-spezifischen Antikörpern (Anti-HCV) das dort genannte Antigen C-100-3 in der ELISA-Ausführung verwendet. Das gentechnologisch in Hefezellen exprimierte Konstrukt C100-3 aus dem NSP 3/4-Bereich umfaßt 363 Aminosequenzen, deren Sequenz in Fig. 1 dargestellt wird, wobei das Numerierungssystem dem der o.g. Patentschrift entspricht.

Die mit dem ELISA-Verfahren bei der Bestimmung von Anti HCV in Proben humanen Ursprungs maximal erzielbare Sensitivität wird jedoch bei chronischen NANB-Patienten mit ca. 80 % und bei akuten NANB-Patienten mit ca. 30 % angenommen. Diese Befunde an humanem Patientenblut werden gestützt durch entsprechende Untersuchungen bei Schimpansen, die mit NANBV infiziert wurden.

Dort gelingt ein positiver Anti HCV-Nachweis auf Basis des C-100-3-Konstruktes erst ca. 6 - 18 Wochen nach stattgefundener ALT-Erhöhung, was als Krankheitssymptom wiederum 3 - 10 Wochen nach Inokulation des NANBV beobachtbar ist. Damit ergeben sich 9 - 26 Wochen nach Infektion eines Schimpansen als Gesamtzeitraum, bis mit dem Immunoassay gemäß dem Stand der Technik mit dem C-100-3-Konstrukt HCV spezifische Antikörper nachweisbar sind.

In der WO 90/11 089 sind weitere Aminosäuresequenzen des HCV beschrieben, die zum Nachweis von Anti HCV verwendet werden können. Jedoch werden keine Angaben zur diagnostischen Relevanz bestimmter Proteinabschnitte gemacht, noch finden sich Beispiele für immundominante Epitope.

Grundsätzliches Problem beim Nachweis von Anti HCV mit den aus der Literatur bekannten Verfahren sind die immer noch bestehenden falsch-positiv und falsch-negativ reagierenden Proben. Die falsch-positiven Proben können aber bis zu 40 % in der Gruppe gesunder Blutspender betragen (WEINER A., et al. Lancet 1990, Vol. 336, S. 695). Aufgrund des Fehlens eines spezifischen und sensitiven HCV-Tests wird daher ein nicht unerheblicher Anteil falsch reaktiver Proben im Blutspendewesen fälschlicherweise verworfen und gleichzeitig tatsächlich anti HCV-positive Patienten immer noch nicht zuverlässig erfaßt.

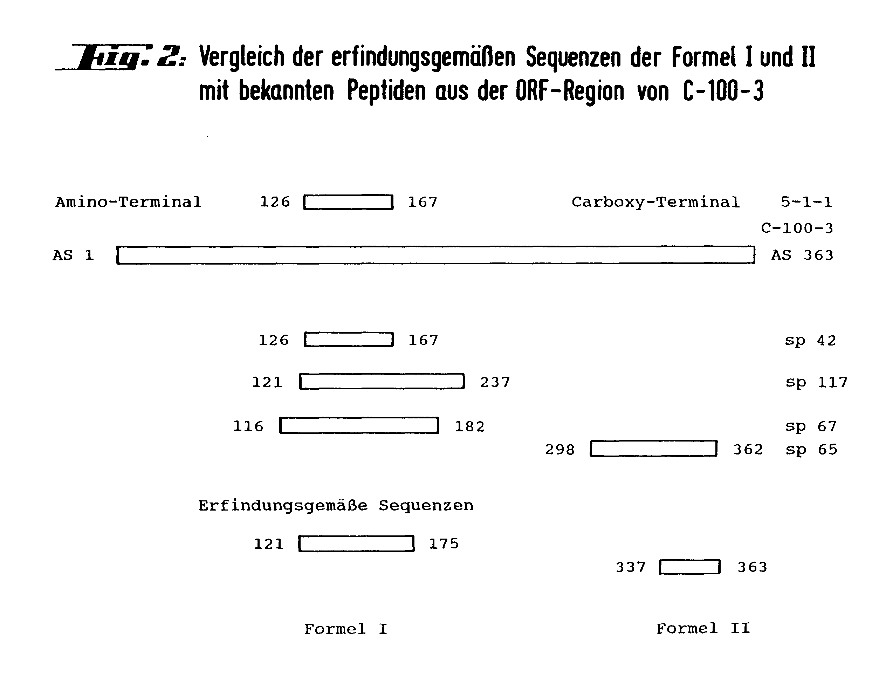

Um diese Nachteile zu beseitigen, wurden von anderen Arbeitsgruppen bestimmte Sequenzen der ORF-Region von C-100-3 (aus WO 89/04 669, Fig. 1) ausgewählt und in einem Immunoassay eingesetzt (Peptide sp42, 117, 67 und und 65, Fig. 2). Bei der Untersuchung von NANB-infizierten Schimpansen konnte keine Verbesserung der diagnostischen Wertigkeit erzielt werden. Die dabei gemachten Beobachtungen führten vielmehr zu dem Schluß, daß keines der beschriebenen Peptide geeignet ist, als Basis einer zuverlässigen IgG- oder IgM-Bestimmung zu dienen, da die Empfindlichkeit unzureichend ist und die diagnostische Lücke von mindestens 7 bis 17 Wochen bis zum Antikörper-Nachweis gegenüber herkömmlichen Nachweismethoden keine Verkürzung bedeutet. Darüber hinaus wurde gefunden, daß insbesondere das sp42 mit einer 20 bis 40 Wochen dauernden diagnostischen Lücke ungeeignet für einen diagnostischen Test ist (SAFFORD J., et al. Int. Symp. on Viral Hepatitis and Liver Disease 1990 Houston, USA Abstract No. 370).

Das als sp67 bezeichnete Polypeptid jedoch soll immundominant sein, obwohl mit diesem Peptid lediglich eine 86%ige Erfassung von anti HCV-positiven Proben möglich war (DAWSON, G.J., et al. VIII th Int. Congress of Virology 1990, Berlin, FRG).

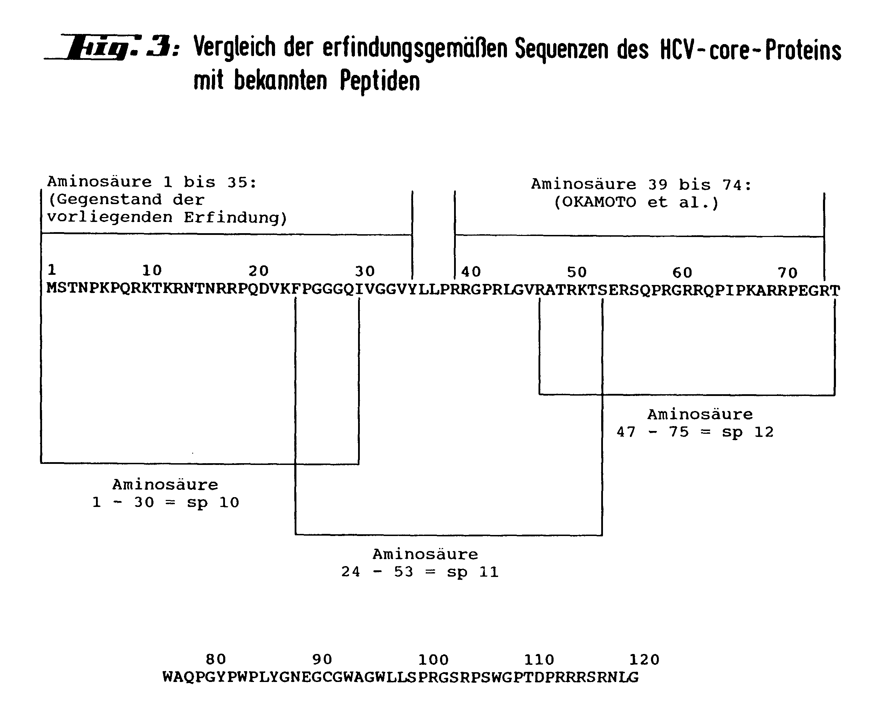

Eine Verbesserung soll mit synthetisch hergestelltem core-Peptid erzielt worden sein (OKAMOTO H., et al. Japan. J. Exp. Med. 1990, Vol 60, No. 4, S. 223 - 233). Dabei wurde von den insgesamt 3 potentiellen Antikörper-Bindestellen der core-Aminosäuresequenz 1-120 (OKAMOTO, H. et al., Japan. J. Exp. Med. 1990, Vol. 60, No. 3, S. 167 - 177) mit Hilfe physiko-chemischer Voraussage-Kriterien nach HOPP and WOOD (Proc. Nat. Acad. Sci. 1981, Vol. 78, S. 3824 - 3828) eine Sequenz ausgewählt. Dieses in Fig. 3 wiedergegebene Peptid umfaßt 36 Aminosäuren der Numerierung No. 39 bis No. 74.

Im Gegensatz zum Anti C-100-Test gelang mit einem auf diesem core-Peptid basierenden ELISA in einigen anti-HCV-positiven Seren ein Nachweis von HCV-Antikörpern.

Dieser Befund wurde durch eine vergleichende Untersuchung mit Hilfe der Polymerase Chain Reaction (PCR) zum Nachweis von HCV-RNA bestätigt.

Jedoch machen gerade die bei einer Untersuchung von 606 Blutspendern ermittelten 26 anti-core HCV-positive Proben (4,3 %) deutlich, daß nur 10 (1,65 %) Fälle mit PCR bestätigt werden konnten. Umgekehrt reagierten 16 Spender (2,65 %) falsch positiv mit diesem Peptid. Das genannte core-Peptid soll daher zwar in einigen Fällen für ein anti-HCV-Nachweisverfahren besser geeignet sein als der Anti C-100-Test, jedoch sind im allgemeinen erhebliche Störanfälligkeiten mit dem core-Peptid des Standes der Technik verbunden.

Der vorliegenden Erfindung lag nun die Aufgabe zugrunde, einen Test zu entwickeln, mit dem man HCV-Infektionen möglichst frühzeitig und mit hoher Spezifität nachweisen kann.

Überraschend wurde nun gefunden, daß bestimmte Polypeptide aus dem Bereich der ORF-Region von C-100-3 für den Nachweis HCV-spezifischer Antikörper besonders geeignet sind und eine deutliche Empfindlichkeitssteigerung gegenüber Peptiden des Standes der Technik bei gleichzeitig signifikant erniedrigter Störanfälligkeit durch unerwünschte falsch positive Reaktionen erzielt werden kann.

Ferner wurde gefunden, daß mit den erfindungsgemäßen Peptiden eine hohe Antigenkonzentration und damit eine hohe Epitopdichte in einem immunchemischen Nachweisverfahren erreicht wird. Dies ist insbesondere dann möglich, wenn durch die Abwesenheit störender Kontaminationen die Untersuchung stark konzentrierter Patientenproben möglich ist.

Es war auch völlig überraschend, daß die erfindungsgemäßen Peptide immundominante Epitope für frühe und späte HCV-Antikörper tragen und daher besonders vorteilhaft für Reihenuntersuchungen eingesetzt werden können.

Ein Gegenstand der Erfindung sind daher Peptide oder Peptidmischungen, die spezifisch mit Antikörpern gegen eine HCV-Infektion reagieren, wobei ihre Aminosäuresequenzen Teile der ORF-Region von C-100-3 und/oder der amino-terminalen HCV core-Region enthalten.

Die Ausdrücke Peptide und Polypeptide werden im Sinne der Erfindung als gleichwertig für Peptide mit bis zu etwa 80 AS verwendet.

Die erfindungsgemäßen Peptide umfassen vorzugsweise Aminosäure-Sequenzen von AS 121 bis AS 175 (Formel I) sowie von AS 337 bis AS 363 (Formel II) des als C-100-3 beschriebenen Genomabschnittes von HCV mit

folgenden Sequenzen:

```
121                                                              175
SGKPAIIPDREVLYREFDEMEECSQHLPYIEQGMMLAEQFKQKALGLLQTASRQA I

337                      363
HVGPGEGAVQWMNRLIAFASRGNHVSP                                      II
```

**Bevorzugte Polypeptide sind:**

<u>4083:</u>
```
121                                                              175
 SGKPAIIPDREVLYREFDEMEECSQHLPYIEQGMMLAEQFKQKALGLLQTASRQA
```
<u>4074:</u>
```
121                                          160
 SGKPAIIPDREVLYREFDEMEECSQHLPYIEQGMMLAEQF
```

<u>4073:</u>

128                                  160

PDREVLYREFDEMEECSQHLPYIEQGMMLAEQF


<u>4072:</u>

136                    160

EFDEMEECSQHLPYIEQGMMLAEQF


<u>4071:</u>

144          160

SQHLPYIEQGMMLAEQF


<u>4081:</u>

161        175

KQKALGLLQTASRQA

<u>4082:</u>

144                        175

SQHLPYIEQGMMLAEQFKQKALGLLQTASRQA


<u>4090:</u>

135                          164

REFDEMEECSQHLPYIEQGMMLAEQFKQKA


<u>4056:</u>

121          135

SGKPAIIPDREVLYR


<u>4055:</u>

129          143

DREVLYREFDEMEEC


<u>4054:</u>

137          151

FDEMEECSQHLPYIE

**4053:**

145                  159

QHLPYIEQGMMLAEQ

**4052:**

153                  167

GMMLAEQFKQKALGL

Für den Nachweis später Antikörper einer HCV-Infektion haben sich insbesondere Sequenzen aus dem Carboxy- und Aminoterminalen Bereich der Formel I als günstig erwiesen, wie z. B. die Peptide 4081, 4056, 4055 sowie das nachstehende 4060:

**4060:**

121                          139

SGKPAIIPDREVLYREFDE

Es wurde auch gefunden, daß der mittlere Bereich der Formel I für die frühe Erfassung von Anti HCV relevant ist. Bevorzugt sind hier die Peptide 4071, 4053 und 4052.

Auf dem Peptid gemäß Formel I wurden weiterhin 3 Epitope lokalisiert, von denen eines späte Antikörper (S) und die anderen frühe Antikörper (F1 und F2) erkennen. Beispielsweise sind Peptide mit einer der folgenden Aminosäuresequenzen bevorzugt:

$$AS_{a1}\text{-DREVLYR-}BS_{b1} \qquad (S)$$

$$AS_{a2}\text{-QHLPYIE-}BS_{b2} \qquad (F1)$$

$$AS_{a3}\text{-KQKALGL-}BS_{b3} \qquad (F2)$$

wobei AS und BS eine beliebige Aminosäure bedeuten und a1 - a3 und b1 - b3 jeweils unabhängig voneinander ganze Zahlen größer oder gleich Null sind und $AS_{a1}$, $BS_{b1}$, $AS_{a2}$, $BS_{b2}$, $AS_{a3}$ und $BS_{b3}$ nicht mit der entsprechenden Sequenz des C-100-3 Genomabschnitts von HCV übereinstimmen.

Im allgemeinen ist es vorteilhaft, Peptide, verknüpfte Peptide oder Peptidmischungen, die für frühe und späte HCV-Antikörper spezifisch sind, zu verwenden, da mit beiden Typen von Bindungsstellen sowohl Proben aus frühen als auch Proben aus späten Infektionsphasen erfaßt werden können. Auch können Serokonversionen, die durch HCV-spezifische IgG- und/oder IgM-Antikörper verursacht werden, sicher erfaßt werden, und die Diskriminierung von positiven und negativen Proben mit außerordentlich hoher Trennschärfe ist im allgemeinen möglich. Zudem gibt es im allgemeinen keine Interferenzen durch das für eine gentechnologische Proteindarstellung erforderliche Expressionssystem oder andere bei der Viruszucht unvermeidliche Wirtszell-Kontaminationen. Auch ist in Seren, Citrat-, Heparin-, und EDTA-Plasmen humanen Ursprungs eine sichere Bestimmung von HCV-spezifischen Antikörpern im allgemeinen gewährleistet und eine Inaktivierung der Proben über ca. 60 Minuten bei ca. 56 °C führt im allgemeinen zu keinen falsch-positiven Resultaten. Schließlich können durch diese erfindungsgemäßen Peptide spezifische Antikörper hergestellt werden, mit deren Hilfe durch die immunchemische Bestimmung entsprechender Antigene im zellfreien Patientenblut die diagnostische Lücke weiter geschlossen werden kann.

Gemäß dem Gegenstand der Erfindung wird eine Serie von neuen Peptiden beschrieben, die vorzugsweise HCV-spezifische Antikörper von rekonvaleszenten oder chronisch infizierten Patienten und/oder HCV-Antikörper von akuten Infektionsphasen erfassen und es werden Polypeptide bzw. Mischungen von Peptiden beschrieben, die sich als Basis von Screeningstests zum nichtdifferenzierenden, aber hochsensitiven und wenig störanfälligen Nachweis von Anti-HCV-Antikörpern eignen.

Die genannten immunoreaktiven Peptide können synthetisch oder gentechnologisch, vorzugsweise synthetisch nach dem Fachmann bekannten Verfahren hergestellt werden. Die chemische Synthese der Peptide kann beispielsweise nach BARANI, G. und MERRIFIELD, R.B. in "The Peptides,

Analysis, Synthesis and Biology", Vol. 2, Academic Press 1980, Ed. Erhard Gross, Johannes Meyenhofer, insbesondere als ein Polypeptid oder als Mischung mehrerer kleiner Peptide mit überlappender oder nicht überlappender Aminosäure-Sequenz hergestellt werden.

Unter die gentechnologisch hergestellten Polypeptide fallen auch Fusionsproteine, deren Fusionsanteil nachträglich abgespalten wurde. Auch fallen Polypeptide darunter, die gegebenenfalls beispielsweise durch Glykosilierung, Acetylierung oder Phosphorylierung modifiziert wurden.

Diese Aminosäure-Sequenzen können sowohl als ein Polypeptid, als auch als Mischung mehrerer kleiner Peptide mit überlappender oder nicht überlappender Aminosäure-Sequenz synthetisiert werden.

Es wurde auch gefunden, daß für einen immunchemischen Anti HCV-Nachweis Mischungen einzelner Peptide bessere diagnostische Eigenschaften haben können, als einzelne Peptide der genannten Strukturen. Besonders vorteilhaft ist es, wenn man Mischungen aus Peptiden der ORF-Region von C-100-3 und der core-Region verwendet.

Häufig ist es vorteilhaft, daß Peptide vielfältig derivatisiert werden, wie z. B. durch Amino-terminale bzw. Carboxy-terminale Angliederung einer oder mehrerer Aminosäuren, vorzugsweise Cystein, um beispielsweise das Verknüpfen von Peptiden untereinander oder an einen Träger zu erreichen, Thioglykolsäure-Amidierung, Carboxyterminale Amidierung, wie z. B. mit Ammonium oder Methylamin. Derartige Modifikationen können die Nettoladung des Polypeptids verändern und die physikochemische Eigenschaften des Peptids verbessern oder die kovalente Bindung des Peptids an einem festen Träger, an Carrier-Proteine oder an ein anderes Peptid erleichtern. Dem Fachmann ist auch bekannt, daß die erfindungsgemäßen Peptide auch untereinander bzw. mit sich selbst gentechnologisch oder synthetisch derart hergestellt werden können, daß mehrere immunrelevante Epitope auf einem Peptid vorliegen.

Herstellbar sind daher Peptide mit einer durch Ersatz, Hinzufügen oder Weglassen von einer oder mehreren Aminosäuren modifizierten erfindungsgemäßen Aminosäuresequenz.

Im allgemeinen führen solche Modifikationen nicht zu direkten Veränderungen der Immunreaktivität eines Peptides, allerdings können durchaus verbesserte immunologische Eigenschaften der Peptide erzielt werden. So neigt z. B. Methionin zu spontaner Oxidation, was durch Austausch gegen Norleucin verhindert werden kann, ohne daß sich die antigenen Eigenschaften des Polypeptides im wesentlichen ändern.

Es ist dem Fachmann bekannt, daß gegebene Aminosäure-Sequenzen den verschiedensten Veränderungen unterworfen werden können, wie z. B. Deletionen, Insertionen oder Substitutionen, womit verschiedene Vorteile verbunden sein können. Solche Modifikationen betreffen z. B. Kombinationen wie Gly, Ala; Val, Ile, Leu; Asp, Glu; Asn, Gln; Ser, Thr; Lys, Arg; Phe, Tyr; Ala, Ser; Ala, Thr; Ala, Val; Ala, Pro; Ala, Glu; Leu, Gln; Gly, Phe; Ile, Ser und Ile, Met.

Ebenfalls kann es vorteilhaft sein, in Form des Zusatzes einer hydrophoben Sequenz, umfassend etwa 2 bis 20 hydrophobe Aminosäuren wie z. B. Phe Ala Phe Ala Phe, die Adsorptionseigenschaften des Polypeptides zu verbessern.

Unter einem immunchemischen Nachweis werden im allgemeinen Verfahren zusammengefaßt, die als homogene (in Lösung) oder heterogene (mit fester Phase) Methoden die Bestimmung von Antigenen und/oder Antikörpern erlauben. Beispiele für diese auch sogenannten Immunoassays sind Enzymimmunoassay (ELISA oder EIA), Radioimmunoassay (RIA), Immunfluoreszenzassay (IFA), Radioimmunopräzipitationsassay (RIPA) oder Agar Gel-Diffusionsassay usw.

Diese zahlreichen, sehr verschiedenen Methoden unterscheiden sich in speziellen Ausführungsformen, in dem zur Detektion verwendeten Marker oder Meßprinzip (z. B. photometrisch, radiometrisch, visuell bzw. per Aggregations-, Streulicht- oder Präzipitations-Verhalten) und in den Festphasen. Dem Fachmann ist es bekannt, daß eine Trennung von gebundenen und freien Proben-Antikörpern oder -Antigen zwar weit verbreitet aber nicht unbedingt erforderlich ist, wie z. B. bei sogenannten homogenen Assays. Bevorzugt sind heterogene Immunoassays, insbesondere heterogene ELISA-Verfahren.

Ebenso ist dem Fachmann bekannt, daß der Begriff "Bestätigungstest" nur die Anwendung eines Immunoassays beschreibt ähnlich wie die als Dot-Verfahren bezeichneten Tests per Name nur beschreiben, auf welche Art das Antigen immobilisiert wird.

Bei dem Antikörper-Nachweis setzt ein immunchemisches Nachweisverfahren während des Ablaufes den Kontakt der Probe mit den beschriebenen Peptidsequenzen voraus, um in einem bestimmten Schritt des jeweiligen Verfahrens einen Antigen-Antikörperkomplex auszubilden oder bei Kompetitions- und Inhibitionstests die Bildung desselben durch Zusatz von geeigneten markierten Reagenzien zu verhindern.

In den direkten Verfahren können die Antikörper mit festphasengebundenen Peptiden oder mit markierten Peptiden oder mit beiden in Kontakt gebracht werden, wobei es unerheblich ist, ob das zugrundeliegende Verfahren als 1-, 2- oder Mehr-Schritt-Methode auf dem Prinzip des 2. Antikörpertests oder dem immunometrischen Testaufbau (Doppel-Antigen-Sandwich) entweder mit gleichen oder unterschiedlichen Peptiden (bzw. Peptidmischungen) auf der Festphase und als Flüssigreagenz für die Detektion sowie in Verbindung mit spezifischen sogenannten Capture-Antikörpern (z. B. Anti IgM) oder Affinitätsreagenzien (z. B. Protein A) beruht.

Die Bindung der Peptide an die Festphase kann kovalent, adsorptiv oder mittels spezifischer Antikörper oder ähnlich affiner Methoden, z. B. über den Biotin/Avidin-Komplex stattfinden, vorzugsweise jedoch adsorptiv.

Als Trägermaterial für die Festphase sind Kunststoffe wie Polystyrol, Polyvinylchlorid, Polyamid und andere synthetische Polymere, natürliche Polymere wie Zellulose sowie derivatisierte natürliche Polymere wie Zelluloseacetat und Nitrozellulose als auch Glas, insbesondere als Glasfaser, geeignet.
Bevorzugt ist als Trägermaterial Polystyrol.

Die Träger können die Form von Kugeln, Stäben, Röhrchen und Mikrotiterplatten haben oder in Form von Suspensionen wie z. B. Latexpartikel vorliegen. Flächenförmige Gebilde wie Streifenpapiere, Plättchen und Membranen sind ebenfalls geeignet. Die Oberfläche der Träger kann sowohl für wässrige Lösungen durchlässig als auch undurchlässig sein.

Bevorzugte Träger sind Kugeln, Röhrchen, Näpfchen, Mikropartikel, Streifenpapiere und Membranen. Besonders bevorzugte Träger sind Mikrotitrationsplatten, Latexpartikel, Polystyrol-Kugeln oder magnetisch anziehbare Teilchen.

Die Peptidkonzentration bei der Beschichtung des Trägers beträgt im allgemeinen ca. 0,01 - 20 µg/ml, vorzugsweise 0,01 - 10 µg/ml, besonders bevorzugt von 2 - 10 µg/ml. Besonders vorteilhaft ist die Verwendung von synthetisch hergestellten Polypeptiden, deren hohe Reinheit und starke Antigenität die Verwendung kleinster Mengen von beispielsweise 0,01 - 2,0 µg/ml, vorzugsweise 0,1 - 0,5 µg/ml gestattet. Die Bindekapazität des Trägers, insbesondere bei Verwendung von Polystyrol, ist im allgemeinen nicht gesättigt, so daß normalerweise mit mehreren verschiedenen Polypeptiden, insbesondere mit 2 - 5, vor allem mit 3 - 4 verschiedenen Polypeptiden, beschichtet werden kann, was von besonderem Vorteil ist.

Bei Verwendung der Peptide als markierte Derivate zur Detektion kommen alle dem Fachmann bekannten Kopplungstechniken in Frage. Auch mehrstufige Verfahren wie z. B. präformierte Peptid-Antikörper-Komplexe, in denen der Antikörper die Markierung trägt, oder hochaffine Systeme, wie z. B. Biotin/Avidin mit Markierung eines dieser Reaktionspartner, können angeordnet werden.

Als Marker können beispielsweise radioaktive Isotope, fluoreszierende oder chemilumineszierende Farbstoffe verwendet werden. Auch Enzyme, die z. B. durch chromogene, luminogene oder fluorogene Substratsysteme nachgewiesen werden oder durch nachgeschaltete Verstärkersysteme mit einem zweiten Enzym, das durch das erste aktiviert wird, können als Marker verwendet werden.

Vorzugsweise werden als Marker Enzyme, insbesondere die alkalische Phosphatase und/oder die Meerrettich Peroxidase oder Chemoluminogene, wie z. B. Acridinium-Ester verwendet.

Die Markierung erfolgt nach Verfahren, die für die genannten Marker im Stand der Technik beschrieben sind.

Im Falle der Markierung der Antikörper mit Peroxidase kann die Periodat Technik nach NAKANE et al., 1974, J. Histochem. Cytochem. 22, 1084 - 1090, angewendet werden oder ein Verfahren gemäß ISHIKAWA et al., 1983, J. Immunoassay 4, 209 - 327, in dem die Partner mit einem heterobifunktionellen Reagenz verknüpft werden.

Neben diesen Verfahren können die Peptide zur Sensibilisierung von geeigneten Oberflächen wie z. B. Latex oder Erythrozyten verwendet werden, um durch Peptid-spezifische Antikörper induzierte, physikochemische Veränderungen wie z. B. Präzipitationen, Aggregation oder Lichtstreuung automatisch oder visuell zu messen. Es ist bekannt, daß die Peptide auch nicht derivatisiert zur Inhibition dieser Meßprinzipien wie auch der zuvor genannten Methoden eingesetzt werden können.

Für den Nachweis der Antigene können immundiagnostische Verfahren verwendet werden, die sich polyklonaler oder monoklonaler Antikörper bedienen, welche mit Hilfe der erfindungsgemäßen Peptide oder Derivaten davon hergestellt werden. Die für das Nachweisverfahren in Frage kommenden Ausführungsformen sind dem Fachmann bekannt und dadurch gekennzeichnet, daß in einem bestimmten Schritt Antikörper-Antigen-Komplexe gebildet werden oder die Komplexbildung in Kompetitionsverfahren durch Zusatz eines markierten Antigens inhibiert wird.

Für die Etablierung eines Antigentests kommen als Festphasen, Marker oder Meßprinzip alle bei der entsprechenden Antikörper-Bestimmung erläuterten Möglichkeiten in Frage, wobei das Kompetitionsprinzip und die Doppelantikörper-Sandwich Technik als immunchemische Methode besonders bevorzugt sind. Dabei ist es unerheblich, ob die Verfahren als 1-, 2- oder 3-Schritt-Verfahren ausgelegt sind. So können Mehrschrittverfahren mit unmarkierten Nachweisantikörpern durchgeführt werden, die mit Hilfe eines weiteren dagegen gerichteten Antikörpers bestimmt werden, der entsprechend markiert ist. Für die Erzeugung der Antikörper ist es vorteilhaft, die Peptide derart zu modifizieren, daß deren immunogene Eigenschaft verbessert wird, wie dies z. B. durch Kopplung am Serumalbumin oder keyhole limpet hemocyanin möglich ist (B.S. Schaffhausen in Hybridoma Technologie in the Biosciences and Medicine, ed. T.A. Springer, Plenum Press NY, London, 1985).

Schließlich kann die vorliegende Erfindung auch angewendet werden bei Verwendung eines immundiagnostischen Elements, das die Festphase und in trockener Form einen Teil oder auch alle erforderlichen Reagenzien enthält, wobei auch hier die neuen Peptide entweder festphasenseitig oder im Detektionsreagenz oder in beiden enthalten sind und eine Antikörper-Bestimmung, einen Antigen-Nachweis oder Kombinationen mit anderen Analyten durchgeführt wird.

Ein unerwarteter Vorteil der neuen Peptide der vorliegenden Erfindung ist, daß sie eine zuverlässige Bestimmung von HCV-Antikörpern gestatten. Ferner werden mit Hilfe dieser Peptide auch frühe Antikörper aus akuten Infektionsphasen erfaßt, was wesentlich die Sensitivität der auf diesen Peptiden beruhenden Nachweise gegenüber dem Stand der Technik verbessert und außerdem die Differenzierung zwischen akuten Phasen einerseits und chronischen bzw. rekonvaleszenten Stadien andererseits dann gestattet, wenn die Peptide mit den entsprechenden Bindungsstellen für diese Antikörper-Typen (frühe bzw. späte Antikörper) getrennt voneinander in zwei verschiedenen Testverfahren verwendet werden.

Schließlich ergibt sich als weiterer Vorteil die hohe Spezifität der Peptide der vorliegenden Erfindung, was zu einer Minimierung der Zahl von falsch-positiv reagierenden Proben führt.

Im Hinblick auf die Virussicherheit im Blutspendewesen einerseits sowie aus Kostengründen andererseits, wurden sogenannte Kombinationstests entwickelt, die seit 1989 verfügbar einen gleichzeitigen nicht-differenzierenden Nachweis von Anti HIV 1 und Anti HIV 2 gestatten (K. Körner et al., Lab.Med. 14, 159 - 161,1990). Möglich war diese Entwicklung durch die hohe Ähnlichkeit, die beide HIV-Subtypen zueinander aufweisen, die zudem der gleichen Virus-Klasse angehören. Damit stützt sich die Anti HIV 1-Bestimmung in derartigen Anti HIV 1/2-Kombinationstesten auch auf eine Kreuzreaktivität von Anti HIV 1 mit HIV 2-Antigenen (M. Busch et al., Transfusion 30/2, 184 - 187, 1990), wie auch umgekehrt der Anti HIV 2-Nachweis durch die stattfindenden Reaktionen von Anti HIV 2 mit HIV 1-Antigenen verstärkt wird. Mit dieser Grundlage stellt sich die Etablierung eines kombinierten Nachweises zweier Antikörper-Spezifitäten dann als verhältnismäßig einfach dar, wenn verwandte bzw. strukturanaloge Antigene verwendet werden.

Der Nachweis eines Erregers der Non A Non B Hepatitis, das sogenannte Hepatitis C Virus (HCV), war die Voraussetzung für die Etablierung einer Anti HCV-Bestimmung gemäß der vorliegenden Erfindung. Trotz der damit verbundenen Verbesserung der Leistungsmerkmale von Screeningtests stellt sich auch mit modernen Anti HCV-Tests erneut das Problem, daß bei der Untersuchung von Einzelspendern mit Anti HIV-Kombinationstests einerseits und mit Anti HCV-Einzeltests andererseits ein erheblicher Aufwand und erhebliche Mehrkosten entstehen.

Allen bisherigen kommerziellen Ausführungen der Anti HCV-Bestimmung sowie der Mehrzahl der Anti HIV-Kombinationstests liegen gentechnologisch hergestellte HCV- bzw. HIV-Polypeptide zugrunde. Es ist jedoch bisher noch nicht gelungen, mehrere verschiedene Antikörper-Spezifitäten in nur einem Testansatz, enthaltend mehrere verschiedene Antigene, gleichzeitig in einem immunchemischen Nachweis zu bestimmen, wenn keine ähnlichen Antigene verwendet werden können, da wie bei HCV (Flavivirus) und HIV (Retrovirus) unterschiedliche Virus-Klassen-Zugehörigkeiten vorliegen. Als verschiedene Antikörper-Spezifitäten werden im Sinne der Erfindung Antikörper verstanden, die keine oder eine sehr geringe Kreuzreaktivität untereiander besitzen. Man ging davon aus, daß ein derartiger Test zum Nachweis von insgesamt drei Antikörper-Spezifitäten gegen mehrere verschiedene virale Antigene hinsichtlich der Empfindlichkeit (Sensitivität) und bezüglich der Störanfälligkeit (Spezifität) infolge gegenseitiger störender Wechselwirkungen eher schlechter ist als die entsprechenden Eigenschaften der jeweiligen Einzeltests.

Überraschenderweise wurde nun auch gefunden, daß mehrere verschiedene Antikörper bzw. Antikörperspezifitäten gegen jeweils verschiedene Pathogene immunchemisch in einem einzigen Test nachgewiesen werden können, wenn verschiedene Epitope von jeweils verschiedenen Pathogenen an einem Träger fixiert sind.

Ferner wurden mit dem erfindungsgemäßen Verfahren Sensivitäten gefunden, die mindestens den Sensivitäten von Einzeltests entsprechen. So wurden z. B. für Anti HIV 1/2 und Anti HCV für das erfindungsgemäße kombinierte Verfahren Empfindlichkeiteigenschaften bestimmt, die denen von Einzeltests mindestens entsprechen.

Es war daher auch völlig überraschend, daß mit dem erfindungsgemäßen Verfahren die Störanfälligkeit durch unerwünschte falsch positive Reaktionen verringert wurde. So wurde beispielsweise bei der Spezifitätsprüfung eines erfindungsgemäßen Anti HIV/ Anti HCV-Tests eine höhere Spezifität erhalten als die Summe beider Einzeltests ergeben würde mit der Folge, daß insgesamt weniger Blutspenden fälschlicherweise verworfen werden müssen.

Für gewisse Fragestellungen, wie z. B. Reihenuntersuchungen, ist es möglicherweise wünschenswert, die gleichzeitige Bestimmung von verschiedenen Pathogenen nicht-differenzierend vorzunehmen. Das bedeutet, daß zwischen den verschiedenen Antikörpern nicht unterschieden wird, sondern nur eine Ja-Antwort oder Nein-Antwort (negativ für alle Antikörperspezifitäten) erhalten wird.

Gegebenenfalls ist es auch wünschenswert, den gleichzeitigen Antikörper-Nachweis differenzierend zu bestimmen. Dies ist im Rahmen der Erfindung ohne weiteres möglich, indem z. B. in einem immunometrischen Test die verwendeten Antigene Virus-spezifisch unterschiedlich markiert werden, wie z. B. HIV 1-Antigene mit Peroxidase, HIV 2-Antigene mit alkalischer Phosphatase und HCV-Antigene mit β-Galaktosidase. Dann können mit verschiedenen Enzym-Substraten die gleichzeitig gebundenen Antikörper-Spezifitäten nacheinander oder gleichzeitig bestimmt werden.

Eine alternative Form der Differenzierung ist die Inhibition einer Antikörper-Spezifität durch den gezielten Zusatz des korrespondierenden Antigens zur Probe.

Als unterschiedliche Pathogene werden hier Pathogene verstanden, gegen die Antikörper von unterschiedlicher Spezifität und im allgemeinen mit keiner oder einer sehr geringen Kreuzreaktivität gerichtet sind. Beispielsweise sind dies HIV 1 + 2, HCV, HTLV I + II, HBV oder Trepanoma pallidum, vorzugsweise HIV und HCV.

Es ist besonders vorteilhaft, Antigene der genannten Pathogene, insbesondere von HIV 1, HIV 2 und HCV, die eine hohe Dichte bzw. Konzentration an Bindungsstellen (Epitope) der entsprechenden Antikörper besitzen an einen Träger zu fixieren. Dadurch können beispielsweise Serokonversionen erfaßt werden, eine Diskriminierung von positiven und negativen Proben mit einer im allgemeinen hohen Trennschärfe ist möglich, eine Interferenz durch das für eine gentechnologische Proteindarstellung erforderliche Expressionssystem kann im allgemeinen nicht eintreten, andere bei Viruszucht unvermeidliche Wirtszell-Kontaminationen liegen im allgemeinen nicht vor, in Seren, Citrat-, Heparin-, und EDTA-Plasmen humanen Ursprungs ist im allgemeinen eine sichere Bestimmung von HIV- und HCV-spezifischen Antikörpern mit unterschiedlicher Spezifität gewährleistet und eine Inaktivierung der Proben über 60 min. bei 56°C führt normalerweise zu keinen Falsch-positiven Resultaten.

Vorzugsweise werden einzelne Epitope und/oder Kombinationen davon, insbesondere von Polypeptiden des HIV 1 und/oder HIV 2 und HCV, die für einen hochsensitiven Anti HIV/Anti HCV-Nachweis geeignet sind, verwendet und ferner geeignete Polypeptid-Mischungen als Basis für einen Screeningtest für einen kombinierten Antikörper-Nachweis.

Ein weiterer Gegenstand der Erfindung sind daher Polypeptidgemische von HIV 1 und/oder HIV 2 und HCV.

Besonders bevorzugt sind folgende Polypeptide:

    1. HIV 1 (Numerierungssystem nach Ratner et al., Nature 1985, 313, 277 - 284):

        IV Transmembranprotein (gp 41): AS 580 - AS 630
        V envelope protein (gp 120): AS 490 - AS 540
        VI core protein ( p 24): AS 240 - AS 390

    2. HIV 2 (Numerierungssystem nach Gyader et al., Nature 1987, 326, 662 - 669):

        VIITransmembranprotein (gp 36): AS 570 - AS 620
        VIII envelope protein (gp 110): AS 480 - AS 530
        IX core protein ( p 26): AS 230 - AS 380

    3. HCV (Numerierungssystem nach WO 89/04669 und WO 90/11089):

        X Nichtstrukturprotein 4 (NSP 4): AS 121 - AS 175
        XI Nichtstrukturprotein 3 (NSP 3): AS 1 - AS 265
        XII Strukturprotein (core) : AS 1 - AS 80

Vor allem bevorzugt sind Mischungen der genannten Polypeptide, insbesondere für einen nicht-differenzierenden Anti HIV/Anti HCV Screeningtest wobei einige beispielhaft beschrieben werden, ohne die vorstellbaren Möglichkeiten darauf zu beschränken:

XIII     gp 41 HIV 1
        gp 36 HIV 2
        NSP 3 HCV

XIV     gp 41 HIV 1
        gp 36 HIV 2
        NSP 4 HCV
        core HCV

XV     gp 41 HIV 1
        gp 36 HIV 2

NSP 3 HCV
NSP 4 HCV
core HCV

XVI     gp 41 HIV 1
        p 24 HIV 1
        gp 36 HIV 2
        NSP 3 HCV
        core HCV

oder

XVII gp 41 HIV 1     p 24 HIV 1
                     gp 36 HIV 2
                     p 24 HIV 2
                     NSP 3 HCV
                     NSP 4 HCV
                     core

Im allgemeinen werden mit den genannten Peptid-Mischungen bessere immunologische Eigenschaften für einen diagnostischen Einsatz erreicht.

Als besonders geeignet haben sich folgende Peptide von HIV 1, HIV 2 und HCV erwiesen:

**XVIII SPH 9  (HIV 1, gp 41):**

586                                              620

RILAVERYLKDQQLLGIWGCSGKLICTTAVPWNAS

**XIX    SPH 20  (HIV 2, gp 36):**

578                                              613

RVTAIEKYLQDQARLNSWGCAFRQVCHTTVPWVNDS

**XX     SP 4083  (HCV, NSP 4):**

121                                              175

SGKPAIIPDREVLYREFDEMEECSQHLPYIEQGMMLAEQFKQKALGLLQTASRQA

oder Teile davon, beispielsweise

XXI    **eine Mischung aus SP 4060 und SP 4082 (HCV, NSP 4):**

121               139

  SGKPAIIPDREVLYREFDE                SP 4060


144                       175

 SQHLPYIEQGMMLAEQFKQKALGLLQTASRQA     SP 4082


XXII  SP 10  (HCV, core):

1                          30

MSTNPKPQRKTKRNTNRRPQDVKFPGGGQI


und/oder


XXIII  SP 31  (HCV, core):

8            24

QRKTKRNTNRRPQDVKF$-\mathrm{NH}_2$


Es ist ebenso möglich, weitere Peptide aus anderen Proteinbereichen von HIV 1, HIV 2 oder HCV zu integrieren, sofern diese Polypeptide immunrelevant sind. Umgekehrt kann es auch, z. B. für epidemiologische Fragestellungen vorteilhaft sein, einen spezifischen Antikörper bei dem nicht-differenzierenden Nachweis durch Weglassen entsprechender Peptide auszuschließen, um z. B. mit einer Mischung aus HIV 1- und HCV-Peptiden nur Anti HIV 1/Anti HCV gleichzeitig zu bestimmen und nicht beispielsweise Anti HBV/Anti HIV 1/Anti HCV. Auch können die mit HCV nicht identischen Peptide, wie z. b. eben HIV, auf analoge Weise, wie oben bei den HCV-Peptiden beschrieben, derivatisiert und modifiziert werden.

Ein wesentlicher Vorteil vorliegender Erfindung ist, daß mehrere Antikörper gegen verschiedene Pathogene in einem einzigen Test nachgewiesen werden können und dabei noch eine so hohe Spezifität bzw. Sensitivität erreicht wird wie mit Einzeltests erreicht werden kann.

Fig. 1 Primärsequenz der Aminosäuren der ORF-Region von C-100-3 (aus WO/89/04669)

Fig. 2 Vergleich der erfindungsgemäßen Sequenzen der Formel I mit bekannten Peptiden aus der ORF-Region von C-100-3

Fig. 3 Vergleich der erfindungsgemäßen Sequenzen des HCV-core-Proteins mit bekannten Peptiden

Die nachfolgend beschriebenen Beispiele stellen Ausführungsformen der Erfindung dar, ohne sie jedoch darauf zu beschränken.

Der Schutzbereich der Anmeldung ist durch die Ansprüche definiert; einige Beispiele, die diesem Schutzbereich nicht entsprechen, dienen lediglich zu Vergleichszwecken.


**Beispiel 1**


**Herstellung von Peptid-Lösungen und Beschichtung von Mikrotrationsplatten mit diesen Peptiden bzw. Peptid-Mischungen**


Aus Stammlösungen der erfindungsgemäßen Peptide in 50 % Essigsäure in destilliertem Wasser, enthaltend 1 bis 10 mg Peptid/ml wurden 2fache Verdünnungsreihen in 0,10 M Natriumbicarbonat pH 9,6 angelegt, also eine Reihe mit den Konzentrationen 50; 25; 12,5; 6,25; 3,12; 1,56; 0,78; 0,39; 0,2; 0,1; 0,05 und 0,01 µg Peptid/ml erhalten. Bei Mischungen von einzelnen Peptiden wurde analog vorgegangen, wobei die Stammlösungen zusätzlich in verschiedenen Verhältnissen gemischt wurden, z. B. 10 : 1 oder 1 : 4, um durch Verdünnung in 0,1 M Natriumbicarbonat die oben angegebenen Gesamt-Endkonzentrationen zu erhalten, die aber bei Mischungen mehrerer Peptide diese gleichkonzentriert (bei 1 : 1-Mischung) oder in verschiedenen Verhältnissen zueinander enthalten.

100 µl einer jeden Verdünnung wurde in jeweils 16 Wells von Mikrotiterplatten, Typ B der Firma Nunc, Roskilde, Dänemark gegeben. Die mit den Verdünnungen gefüllten Testplatten wurden 18 Stunden bei 20°C belassen, dann wurden die Lösungen in den Wells abgesaugt und die Wells 3 - 4 mal mit 300 µl einer Lösung von 10 g/l Rinderserumalbumin in phosphatgepufferter physiologischer Kochsalzlösung, (PBS, pH 7,4) durch Füllen und Absaugen gewaschen und die Testplatten anschließend über Kieselgel bei 20°C getrocknet.

**Herstellung eines Peroxidase-markierten Antikörpers gegen Humanes Immunglobulin der IgG-Klasse (h-IgG) sowie TMD-Substrat für die Detektion**

Antikörper gegen h-IgG wurden gemäß der Methode von KOEHLER und MILSTEIN zur Darstellung monoklonaler Antikörper erzeugt (Nature 256, 495, 1975), wobei verschiedene monoklonale Antikörper mit der gleichen Antigen-Spezifität mit der von STÄHLI et al. (J. of Immunological Methods 32, 297 - 304, 1980) beschriebenen Methode identifiziert wurden.

Nach gelchromatographischer Reinigung und Dialyse gegen Phosphat-gepufferter Saline (PBS, pH 7,4) wurde der die monoklonalen Antikörper-Fraktion enthaltende Pool (4 mg Protein/ml) anschließend mit N-gamma-Maleimidobutylyloxisuccinimid (GMBS), bezogen von der Fa. Behring Diagnostics, ungesetzt wie von TANAMORI et al. (J. Immunol. Meth. 62, 123 - 131, 1983) beschrieben.

2-Iminothiolanhydrochlorid (Fa. Sigman, Kat. Nr. I 6256) wurde mit Meerrettich-Peroxidase (POD), bezogen von der Fa. Boehringer Mannheim, Kat. Nr. 413470, umgesetzt wie von KING et al. (Biochem. 17, 1499 - 1506, 1978) beschrieben. Aus dem GMBS-IgG-Konjugat und dem Iminothiolan-POD Konjugat wurde ein IgG-POD Konjugat hergestellt wie von TANAMORI et al. (supra) beschrieben.

Die erhaltene Lösung des IgG-POD Konjugats hatte einen Proteingehalt von 360 µg/ml. Das Verhältnis von POD zu IgG wurde mit 2.8 bestimmt. Die Lösung wurde anschließend auf 500 ng/ml IgG-POD mit einer Lösung von 50 ml/l foetalem Kälberserum (FKS), 5 g/l Polyoxiethylen-(20)-sorbitan Monalaureat (RTween 20) in PBS verdünnt und erhielt die Bezeichnung Anti IgG/POD-Konjugat. Für die Verwendung im ELISA wurde die nachfolgende Verdünnung in Tris-Puffer pH 7,4, enthaltend 0,5 % RTween 20 variiert (zwischen 1 : 100- und 1 : 20.000-Verdünnung), um einheitlich eine 1 : 26-Endverdünnung in Konjugat-Puffer herzustellen, enthaltend 0,1 M 2-Amino-2(hydroxymethyl) 1,3-propandiol (Tris), 0,1 M Kochsalz (NaCl) sowie 0,1 % RTween pH 8,4.

Gemäß dem Stand der Technik dargestellte polyklonale Antikörper vom Kaninchen wurden so eingestellt, daß ebenfalls eine Gebrauchsverdünnung von 1 + 25 erzielt wurde.

Zum Nachweis von Anti-IgG/POD-Konjugat wurde ein Substratsystem oder eine Substratzubereitung verwandt, enthaltend Wasserstoffperoxid und Tetramethylbenzidin (TMB), welche aus zwei Stammlösungen hergestellt wurde.

Stammlösung 1: TMB-Dihydrochlorid wurde unter Rühren in einer Konzentration von 5 g/l, d. h. von 16 mmol/l in bidestilliertem Wasser gelöst und mit 5 normaler Salzsäure auf pH 1,5 eingestellt. Zu dieser Lösung wurde Penicillin G unter Rühren in einer Endkonzentration von 200 mg/l, d. h. von 0,56 mmol/l zugesetzt.

Stammlösung 2: zu 900 ml bidestilliertem Wasser wurde 1,4 ml Eisessig, 1,5 ml 1 normale NaOH und 250 mg, d. h. 3 mmol $H_2O_2$ als Harnstoff-Wasserstoffperoxid-Addukt zugesetzt. Nach vollständigem Lösen wurde mit bidestilliertem Wasser auf 1 l aufgefüllt.

TMB-Substratzubereitung: Ein Volumenteil der Stammlösung 1 und 10 Volumenteile der Stammlösung 2 wurden miteinander vermischt.

**Beispiel 3**

**Stand der Technik**

Beispielsweise wurde ein kommerziell erhältlicher ELISA Testkit (HP1) eingesetzt, in dem als Antigen das in der WO 89/04669 beschriebene, gentechnologisch in Hefe hergestellte C-100-3 Konstrukt verwendet wird. Bei der Untersuchung von humanen Seren und Plasmen wurde, wie in der Packungsbeilage des Herstellers angegeben gearbeitet, z. B. Probenverdünnung von 1 : 11 und 1 h-Inkubation von Serum, 1 h-Inkubation von Konjugat Anti human IgG/POD sowie dem Enzymsubstrat-System mit o-Phenylendiamin (OPD) als Substrat, photometrischer Messung bei 492 nm sowie die Grenzwert-Festlegung (zu dem Mittelwert der Negativ-Kontrollen wird ein threshold von 0,40 E addiert).

Ganz analog wurde mit einem weiteren kommerziell erhältlichen ELISA (HP 2) gearbeitet, der neben dem C-100-Konstrukt zusätzlich Epitope aus dem core-Bereich sowie aus dem NSP 3-Bereich (c33c) enthält, das heißt, daß bei der Untersuchung humaner Proben der Originaltestkit unter Beibehaltung aller vom Hersteller angegebenen Durchführungsangaben - wie oben beschrieben - angewendet wurde.

Demgegenüber wurde bei der Bestimmung von HCV-spezifischen Antikörpern in Schimpansen-Serum oder -Plasma mit beiden Handelsprodukten derart verfahren, daß mit einem am Kaninchen erzeugten polyklonalen Antikörper gegen Human IgG detektiert wurde. Dazu wurde die IgG-Fraktion des Kaninchen-Antiserums wie in Beispiel 2 beschrieben gereinigt, dialysiert und mit Peroxidase (POD) markiert. Die Endkonzentration wurde im Vergleich zum monoklonalen Anti-IgG/POD-Konjugat etwa 4fach konzentrierter eingestellt, um über die in Vorversuchen validierte Kreuzreaktion der Antikörper gegen humanes IgG für Schimpansen-IgG sicher zu gestalten.

Die Grenzwert-Festlegung mußte modifiziert werden, wie in Tab. 10 A - C beschrieben, da die Initialwerte aller Initialwerte aller Schimpansen bereits höhere Werte aufwiesen (siehe Tab. 10 A - C).

Bei dem vergleichenden Anti HIV 1 + 2-Kombinationstest zur nicht-differenzierenden Bestimmung von HIV 1- und HIV 2-Antikörpern handelt es sich um ein kommerziell erhältliches Produkt (HP 3), das auf synthetisch hergestellten Peptiden von HIV 1 und HIV 2 beruht. Auch bei diesem Test wurde gemäß der Packungsbeilage des Herstellers gearbeitet, z. B. Probenverdünnung 1 : 2, 30 Min.-Inkubation von Serum, 30 Min.-Inkubation von Konjugat (Anti human IgG/POD) sowie dem Enzymsubstratsystem mit Tetramethylbenzidin (TMB) als Substrat, photometrischer Messung bei 450 nm sowie der Grenzwert-Festlegung (zu dem Mittelwert der Negativ-Kontrollen wird ein threshold von 0,250 addiert).

**Beispiel 4**

**Bestimmung von humanen Antikörpern der Immunglobulin G-Klasse gegen HCV im ELISA mit den erfindungsgemäßen Peptiden**

50 µl Serum oder Plasma wurden zu 50 µ l Probenpuffer, enthaltend 0,3 M Tris, 0,3 M NaCl, 20 % Boviserin und 0,1 % $^R$Tween 20 in Wells von Mikrotiterplatten gegeben, die wie beschrieben mit Peptiden bzw. Peptid-Mischungen beschichtet wurden. Nach Inkubation über 30 Min. bei 37°C wurde der Inhalt der Wells durch Absaugen entfernt und die Wells fünfmal mit Waschpuffer enthaltend 1 g/l $^R$Tween 20 in PBS gewaschen. Dann erfolgte die Zugabe von 100 µl endverdünntem Konjugat in die Wells, wobei bevorzugt mit einer Vorverdünnung von 1 : 3000 in Tris-, 0,5 % Tween 20 und einer Endverdünnung von 1 : 26 in Konjugatpuffer gearbeitet wurde. Nach einer Inkubation über 30 Min. bei 37°C wurde der Inhalt der Wells durch Absaugen entfernt und wiederum fünfmal gewaschen. Anschließend wurden in jedes Well 100 µl TMB-Substratzubereitung gegeben, 30 Min. bei 20 - 22°C inkubiert und die Inkubation durch Zugabe von 100 µl 1-normaler Schwefelsäure beendet. Die Extinktion der gefärbten Lösung wurde bei einer Wellenlänge von 450 nm ($E_{450}$) gegen einen Leerwert von PBS gemessen.

Als Anti-HCV-positiv wurden solche Proben eingestuft, die eine $E_{450}$ von größer als 0,10 erzeugten, als Anti HCV-grenzwertig die Proben, deren $E_{450}$ im Bereich von 0,05 bis 0,10 lag und als Anti HCV-negativ solche Proben, die eine $E_{450}$ kleiner als 0,05 erzeugten.

In Tab. 1 werden Resultate zusammengefaßt, die aus der in Beispiel 1, 2 und 4 beschriebenen Bestimmung von humanen Proben mit dem erfindungsgemäßen Peptid 4083 sowie einer Mischung kleinerer Sequenzen der Formel I erhalten wurden. Analog werden die Daten der Tab. 2 mit dem erfindungsgemäßen Peptid SP 10 erhalten, wobei die Ergebnisse beider ELISAs mit denen des HP 1 (Beispiel 3) verglichen wurden.

Tab. 1

| Ergebnisse der Bestimmung von Anti HCV mit einem ELISA, enthaltend das Peptid 4083 sowie einer Mischung kleinerer Peptide auf der Festphase mit humanen Proben | | | | |
|---|---|---|---|---|
| Positiv mit Peptid-ELISA | | | | |
| | | (2 µg 4083/ml) | | (2 µg 4060/ml und 2 µg 4082/ml) |
| | | initial positiv | retest positiv | retest positiv |
| Anti HCV-positive Proben (HP1) | | | | |
| aus Frankreich | n=15 | 15 | 15 | 15 |
| aus Österreich | n=17 | 17 | 17 | 17 |

Tab. 1   (fortgesetzt)

| Ergebnisse der Bestimmung von Anti HCV mit einem ELISA, enthaltend das Peptid 4083 sowie einer Mischung kleinerer Peptide auf der Festphase mit humanen Proben | | | | |
|---|---|---|---|---|
| Positiv mit Peptid-ELISA | | | | |
| | | (2 µg 4083/ml) | | (2 µg 4060/ml und 2 µg 4082/ml) |
| | | initial positiv | retest positiv | retest positiv |
| Anti HCV-positive Proben (HP1) | | | | |
| aus Deutschl. | n=20 | 20 | 20 | 20 |
| aus USA | n=49 | 49 | 49 | 49 |
| Gesamt | n=101 | 101[1] | 101[1] | 101[2] |
| gepaarte Seren/Plasma von gesunden Blutspendern | | | | |
| n = 259 Seren | | 2 | 1 | n.d. |
| n = 259 Plasmen | | 1 | 0 | n.d. |

[1] n = 97 Proben zeigten Extinktionen von >> 2,5 E woraus bei einem Grenzwert von 0,10 E ratios von > 25 resultieren

[2] n = 94 Proben zeigten Extinktionen von > 2,5 E. Nur 4 bzw. 7 Proben reagierten schwächer, aber mit $E_{450}$-Werten zwischen 0,8 und 2,5 und einem Grenzwert von 0,10 E immer noch recht stark.

Tab. 2

| Ergebnisse der Bestimmung von Anti HCV mit einem ELISA, enthaltend das neue Peptid SP 10 auf der Festphase mit humanen Proben | | | |
|---|---|---|---|
| | Positiv mit Peptid-ELISA (2 µg SP 10/ml) | | |
| Anti HCV-positive Proben (HP1) | | initial positiv | retest positiv |
| aus Frankreich | n = 35 | 35 | 35 |
| aus Österreich | n = 26 | 26 | 26 |
| aus Deutschland | n = 29 | 29 | 29 |
| aus USA | n = 53 | 53 | 53 |
| Gesamt | $\overline{143}$ | $\overline{143}$[1] | $\overline{143}$[1] |
| gepaarte Seren/Plasmen (siehe Verteilungskurve von gesunden Blutspendern der Fig. 2) | | | |
| n = 500 Seren | | 0 | 0 |
| n = 500 Plasmen | | 0 | 0 |

1) n = 142 Proben zeigten Extinktionen von > 2,5 E, woraus bei einem Grenzwert von 0,1 E ratios von >> 25 resultieren; nur eine Probe reagierte mit einem Wert von 1,2 E schwächer, aber immer noch wesentlich stärker als bei dem kommerziellen ELISA (HP 1).

Von den geprüften Humanproben, die mit einem kommerziellem Test (HP1) als Anti HCV-positiv klassifiziert waren, wurden alle Proben mit allen ELISAs basierend auf den erfindungsgemäßen Peptiden ebenfalls positiv gefunden. Dabei fällt neben der sehr guten Übereinstimmung zu den Ergebnissen des kommerziellen Tests (HP1) eine sehr starke Signalbildung der Peptid-ELISAs auf. Gleichzeitig machen die Ergebnisse der Reihenuntersuchung von gesunden Blutspendern deutlich, daß die Störanfälligkeit des Tests äußerst gering ist. So wurde bei der Testung von gesunden Spendern von Serum und Plasma eine nur äußerst minimale unspezifische Bindung an die erfindungsgemäßen Peptide beobachtet, d. h. es wurden nur wenige falsch-positiv-Resultate erhalten.

In Tab. 3 sind weitere Resultate wiedergegeben, die durch Testung von humanen Proben mit ELISAs erhalten wurden (nach Beispiel 1,2 und 4), die auf der Verwendung kleinerer Sequenzen (15 Aminosäuren) der Formel (I) beruhen. Es wird deutlich, daß die Peptide zur Epitop-Definition von HCV-Antikörpern bzw. als Mischung von mehrerer Peptiden, bevorzugt 3 bis 6 Peptide enthaltend, für die Bestimmung von Anti HCV geeignet sind.

Tab. 3

Reaktivitäten von humanen Anti HCV-positiven Proben in ELISAs mit kleineren, 15 Aminosäuren umfassende Peptide auf der Oberfläche von Mikrotiterplatten nach Beispiel 1; die Resultate werden als Extinktionswerte bei 450 nm ($E_{450}$) wiedergegeben (- = negativer Befund unter 0,10 E).

| Proben-Nr. | 4056 | 4055 | 4053 | 4052 | 4081 | 4091 | Mixtur 4056 4055 4053 4052 4081 4091 |
|---|---|---|---|---|---|---|---|
| µg/ml | 2 | 2 | 2 | 2 | 2 | 2 | je 0,5 |
| BC90-89 | >2,50 | 0,20 | - | - | - | - | 1,40 |
| 252 | - | - | 0,20 | - | >2,50 | - | 2,10 |
| 90 | 0,80 | - | - | - | 1,60 | - | 2,00 |
| 268 | >2,50 | - | 0,20 | - | >2,50 | >2,50 | >2,50 |
| 84 | - | 1,20 | 1,60 | - | 1,70 | >2,50 | >2,50 |
| 225 | >2,50 | >2,50 | - | - | >2,50 | >2,50 | >2,50 |
| 270 | 2,00 | 1,70 | - | - | 2,20 | >2,50 | >2,50 |
| 229 | 0,20 | - | - | 1,10 | 1,00 | - | 1,50 |
| 288 | >2,50 | >2,50 | - | 0,50 | >2,50 | >2,50 | >2,50 |
| 290 | >2,50 | >2,50 | - | 0,20 | >2,50 | >2,50 | >2,50 |
| 242 | - | 0,50 | - | - | 1,20 | 1,40 | >2,50 |
| 137 | - | - | - | - | 0,40 | 0,35 | 0,70 |
| 286 | - | - | 0,60 | 0,15 | 0,45 | 0,15 | 0,90 |
| 192 | 0,20 | >2,50 | - | 1,00 | - | - | >2,50 |
| 235 | 0,15 | - | 0,40 | - | 0,20 | - | 0,30 |
| 85 | - | - | - | 0,75 | - | - | 0,40 |

Dargestellt sind die Reaktivitäten, wie sie mit einer Mischung von erfindungsgemäßen Peptiden erhalten wurden. Es fällt auf, daß durch die sehr starke Signalbildung der Peptid-ELISA eine zuverlässige Diskriminierung zwischen Positiv- und Negativ-Ergebnissen erzielt wird. Analoge Resultate wurden auch mit folgenden erfindungsgemäßen Peptiden bzw. Peptidmischungen erzielt.

4074/4081 (je 2 µg/ml)

4074/4082 (0,5 und 0,125 µg/ml)

4060/4071/4081 (je 2 µg/ml), wobei sich Mischungen kleinerer, ca. 15 AS umfassender Peptide als durchaus geeignet erwiesen:

4056/4055 und 4052 (je 0,5 µg/ml).

Eine Abhängigkeit der Reaktivität der Proben von deren geographischem Ursprung konnte bei der Testung mit allen erfindungsgemäßen Peptiden in keinem Fall festgestellt werden.

**Beispiel 5**

**Optimierung der ELISA-Bestimmung**

Als veränderliche Parameter der ELISA-Bestimmung wurden hauptsächlich die bei der Beschichtung verwendeten Peptid-Konzentration oder bei Mischungen von Peptiden deren Gesamtkonzentration sowie Verhältnis zueinander variiert bei einer konstanten Konjugat-Konzentration von 1 : 3000 und 1 : 26-Verdünnung. Zusätzlich wurde bei festgelegten Beschichtungs-Konzentrationen die Konjugat-Vorverdünnung variiert. Beide veränderlichen Größen wurden hinsichtlich Spezifität durch Testung von Blutspender-Seren und -Plasmen validiert sowie in Bezug auf Sensitivität durch Bestimmung von Anti HCV in positiven Kollektiven. Darüber hinaus wurde die Grenzempfindlichkeit in Form der analytischen Sensitivität durch serielle Verdünnung von Anti HCV-positiven Proben (1 : 2, 1 : 4 usw. in Anti HCV-negativen Seren) ermittelt und mit den Daten eines kommerziellen Tests ebenso verglichen, wie mit den Resultaten, die unter Verwendung des Literatur-beschriebenen Peptids erzielt wurden.

Die mit humanen Proben erhaltenen Ergebnisse sind in Tab. 4 beispielhaft zusammengefaßt.

Tab. 4

| Vergleichende Titration von Anti HCV-positiven Seren und Plasmen im Peptid-ELISA (4083) und einem kommerziell er- hältlichen Test. Angegeben werden ratios als Quotienten der spezifischen Signale zum Grenzwert, wobei Werte größer 1 ein positives, und Werte von kleiner 1 ein negatives Ergebnis anzeigen. | | | |
|---|---|---|---|
| Serum in neg. | Verdünnung Serum | Peptid-ELISA (4083) 2 µg/ml 4083 Grenzwert 0,10 E | kommerzieller Test (HP1) Grenzwert 0,454 E |
| HC 90-84 | 1: 1 | > 25 | > 6 |
| | 1: 2 | > 25 | > 6 |
| | 1: 4 | > 25 | > 6 |
| | 1: 8 | > 25 | > 6 |
| | 1: 16 | 24 | > 6 |
| | 1: 32 | 10,7 | 4,1 |
| | 1: 64 | 6,1 | 2,0 |
| | 1: 128 | 2,5 | 0,8 neg. |
| | 1: 256 | 1,5 | 0,5 |
| | 1: 512 | 1,1 | 0,5 |
| | 1:1024 | 0,6 neg. | 0,2 |
| HC 90-90 | 1: 1 | > 25 | > 6 |
| | 1: 2 | > 25 | > 6 |
| | 1: 4 | > 25 | > 6 |
| | 1: 8 | > 25 | > 6 |
| | 1: 16 | > 25 | > 6 |
| | 1: 32 | 11,8 | 5,4 |
| | 1: 64 | 5,9 | 3,7 |
| | 1: 128 | 3,3 | 1,4 |
| | 1: 256 | 1,8 | 0,9 neg. |
| | 1: 512 | 1,08 | 0,4 |
| | 1:1024 | 0,5 neg. | 0,3 |
| | 1:2048 | 0,4 | 0,2 |
| HC 90-252 | 1: 1 | > 25 | > 6 |
| | 1: 2 | > 25 | > 6 |
| | 1: 4 | > 25 | 4,5 |
| | 1: 8 | 20 | 3,2 |
| | 1: 16 | 12,2 | 2,7 |
| | 1: 32 | 6,5 | 1,2 |

Tab. 4   (fortgesetzt)

| Vergleichende Titration von Anti HCV-positiven Seren und Plasmen im Peptid-ELISA (4083) und einem kommerziell er- hältlichen Test. Angegeben werden ratios als Quotienten der spezifischen Signale zum Grenzwert, wobei Werte größer 1 ein positives, und Werte von kleiner 1 ein negatives Ergebnis anzeigen. | | | |
|---|---|---|---|
| Serum in neg. | Verdünnung Serum | Peptid-ELISA (4083) 2 µg/ml 4083 Grenzwert 0,10 E | kommerzieller Test (HP1) Grenzwert 0,454 E |
|  | 1: 64 | 4,2 | 0,8 neg. |
|  | 1: 128 | 2,4 | 0,5 |
|  | 1: 256 | 0.97 gw[1] |  |
| HC 90-296 | 1: 1 | > 25 | > 6 |
|  | 1: 2 | > 25 | > 6 |
|  | 1: 4 | 20 | > 6 |
|  | 1: 8 | 8,5 | 5,0 |
|  | 1: 16 | 3,7 | 2,9 |
|  | 1: 32 | 2,9 | 0,7 neg. |
|  | 1: 64 | 1,4 | 0,5 |
|  | 1:128 | 0,8 neg. | 0,4 |
|  | 1:256 | 0,3 | 0,2 |
| HC 90-83 | 1: 1 | > 25 | > 6 |
|  | 1: 10 | 20 | 2,1 |
|  | 1:100 | 6,5 | 0,4 neg. |
| HC 90-240 | 1: 1 | > 25 | > 6 |
|  | 1: 10 | 8 | 1,5 |
|  | 1:100 | 1,4 | 0,3 neg. |
| HC 90-239 | 1: 1 | > 25 | > 6 |
|  | 1: 10 | 7,8 | 2,0 |
|  | 1:100 | 1,4 | 0.1 neg. |
| HC 90-89 | 1: 1 | > 25 | > 6 |
|  | 1: 10 | > 25 | > 6 |
|  | 1:100 | 9 | 3,5 |
| HC 90-137 | 1: 1 | > 25 | > 6 |
|  | 1: 1 | 21 | 4,2 |
|  | 1:100 | 4 | 1,4 |

[1] gw = grenzwertig

Die Ergebnisse der Tab. 4 machen deutlich, daß die Sensitivität des auf den erfindungsgemäßen Peptiden beruhenden ELISAs, gemessen an der Grenzempfindlichkeit von Serumtitrationen mindestens so gut ist wie der vergleichend in identischen Serumverdünnungen getestete kommerzielle Test (HP1). In vielen Fällen wurden sogar verdünnte Proben noch als signifikant positiv mit dem Peptid ELISA gemessen, die im kommerziellen Test (HP1) bereits wiederholbar negativ reagierten, so daß insgesamt eine bessere Nachweisgrenze von HCV-Antikörpern mit den erfindungsgemäßen Peptiden resultiert. Analoge Ergebnisse wurden ebenfalls mit anderen Peptidkonzentrationen, Peptidsequenzen oder Mischungen der neuen Peptide erzielt; wie z. B.

　　4083 (0,25 µg/ml)
　　4074/4081 (je 0,25 µg/ml)
　　4074/4082 (je 0,5 µg/ml)
　　4074/4082 (0,5 und 0,125 µ g/ml)
　　4060/4082 (je 2 µg/ml)

Über die Bestimmung der Grenzempfindlichkeit hinaus wurde bei optimierten Systemen auch die Spezifität anhand Nicht-HCV-spezifischer Antikörper sowie anderer potentieller Störfaktoren überprüft, wobei deutlich wurde, daß die Anti HCV-Bestimmung mit den erfindungsgemäßen Peptiden für HCV spezifisch ist und keinen bekannten Interferenzen wie z. B. Kreuzreaktionen anderer Antikörper, Störungen durch Hitzeinaktivierung o.ä. unterliegt. Sinngemäß gilt dies auch für andere angegebene neue Peptide bzw. Peptidmischungen, bei denen einheitlich eine hohe Serumkonzentration (Verdünnung 1 : 2 in Probenpuffer) angewendet wurde, um die Sensitivität zu steigern, was durch die Reinheit der Peptide und der hohen Antigen-Dichte auf der Festphase möglich wurde.

Die in Tab. 5 zusammengefaßten Vergleiche zwischen einem weiteren erfindungsgemäßen core-Peptid, dem SP10 und HP1 machen ebenfalls deutlich, daß das erfindungsgemäße Peptid gegenüber dem Stand der Technik Vorteile bietet, die wiederum in verbesserten Grenzempfindlichkeiten, d. h. gesteigerter analytischer Sensitivität besteht. So wurden die 4 Titrationen der Tab. 5 gegenüber dem publizierten Peptid um den Faktor 2 bis 4 empfindlicher bestimmt und verglichen mit einem kommerziellen Test (HP1) sogar um den Faktor 8 bis 32.

## Tab. 5

Vergleich der analytischen Sensitivität eines ELISAs basierend auf einem Core-Peptid(Sp10, 2 μg/ml) zum Stand der Technik. Dazu wurden Verdünnungsreihen von Anti HCV positiven humanen Seren in negativem Humanserum hergestellt und die Ergebnisse der Testung mit einem ELISA basierend auf einem erfindungsgemäßen Peptid (SP10) verglichen mit dem kommerziellen HCV- ELISA (HP1) sowie einem Peptid, das gemäß der Abb. 2 in der Literatur als immunrelevant beschrieben wird (OKAMOTO et al.). Alle Angaben stellen Extinktionswerte dar ($E_{450\ nm}$), wobei die als reaktiv anzusehenden Ergebnisse unterstrichen sind.

| Serum No. | erfindungsgemäßes Peptid SP10 | | literaturbekanntes Peptid (OKAMOTO) | | kommerzieller Anti HCV ELISA (HP1) |
|---|---|---|---|---|---|
| Verdünnung | AA 1 - 30 | | AA 39 - 74 | | |
| | 2 μg/ml | 0,25 μg/ml | 2 μg/ml | 0,25 μg/ml | |
| HC 90-85   1:1 | > 2,500 | > 2,500 | > 2,500 | > 2,500 | > 2,500 |
| 1 : 32 | > 2,500 | > 2,500 | 1,008 | 0,878 | <u>0,908</u> |
| 1 : 64 | 1,586 | 0,742 | 0,548 | 0,477 | 0,342  — |
| 1 : 128 | 1,032 | 0,783 | 0,250 | <u>0,183</u> | 0,153  — |
| 1 : 256 | 0,601 | 0,519 | <u>0,101</u> | 0,098 | |
| 1 : 512 | 0,328 | 0,232 | 0,060 | 0,051 | |
| 1 : 1024 | <u>0,188</u> | <u>0,122</u> | | | |
| 1 : 2048 | 0,080 | 0,034 | | | |
| HC 90-225   1:1 | > 2,500 | > 2,500 | > 2,500 | > 2,500 | > 2,500 |
| 1 : 16 | > 2,500 | > 2,500 | > 2,500 | 2,366 | > 2,500 |

EP 0 484 787 B1

**Fortsetzung Tab. 5**

|          |          | > 2,500 | 2,065   | 2,020   | 1,619   |   | > 2,500 |     |
| -------- | -------- | ------- | ------- | ------- | ------- | - | ------- | --- |
| 1 : 32   |          | > 2,500 | 2,065   | 2,020   | 1,619   | . | > 2,500 |     |
| 1 : 64   |          | 1,669   | 1,520   | 0,655   | 0,883   |   | 1,759   |     |
| 1 : 128  |          | 0,971   | 0,790   | 0,414   | 0,544   |   | 0,909   |     |
| 1 : 256  |          | 0,561   | 0,374   | 0,281   | 0,215   |   | 0,380   | –   |
| 1 : 512  |          | 0,486   | 0,191   | 0,146   | 0,157   |   | 0,122   | –   |
| 1 : 1024 |          | 0,295   | 0,141   | 0,080   | 0,115   |   |         |     |
| HC 90-270 | 1:1     | > 2,500 | > 2,500 | > 2,500 | > 2,500 |   | > 2,500 |     |
| 1 : 8    |          | > 2,500 | > 2,500 | > 2,500 | 2,222   |   | 1,902   |     |
| 1 : 16   |          | > 2,500 | 2,500   | 1,478   | 1,016   |   | 0,980   |     |
| 1 : 32   |          | 1,512   | 1,408   | 0,743   | 0,478   |   | 0,501   |     |
| 1 : 64   |          | 0,898   | 0,802   | 0,322   | 0,216   |   | 0,259   | –   |
| 1 : 128  |          | 0,536   | 0,483   | 0,121   | 0,096   |   | 0,179   | –   |
| 1 : 256  |          | 0,312   | 0,275   | 0,050   | 0,041   |   |         |     |
| HC 90-354 | 1: 1    | > 2,500 | > 2,500 | > 2,500 | > 2,500 |   | > 2,500 |     |
| 1 : 32   |          | > 2,500 | > 2,500 | 1,917   | 0,786   |   | 0,496   | +/– |
| 1 : 64   |          | > 2,500 | 1,833   | 1,282   | 0,527   |   | 0,352   | –   |
| 1 : 128  |          | 1,703   | 0,873   | 0,685   | 0,249   |   | 0,200   | –   |
| 1 : 256  |          | 0,918   | 0,410   | 0,367   | 0,082   |   |         |     |
| 1 : 512  |          | 0,582   | 0,310   | 0,145   |         |   |         |     |
| 1 : 1024 |          | 0,326   | 0,178   | 0,072   |         |   |         |     |
| 1 : 2048 |          | 0,159   | 0,084   |         |         |   |         |     |

EP 0 484 787 B1

Zu ähnlichen Ergebnissen führte die Auswertung eines weiteren erfindungsgemäßen core-Peptids (SP 23). Die in Tab. 6 vergleichend dargestellten Ergebnisse lassen erkennen, daß das SP 23 dem SP 10 hinsichtlich der Empfindlichkeit entspricht, wobei sich beide Peptide vorteilhaft von dem Literatur-beschriebenen Peptid abheben.

Obwohl das Literatur-analoge Peptid SP 12 (AS 47-75) nicht exakt dem von OKAMOTO et al. beschriebenen Peptid entspricht (AS 39-74), wird durch die Ergebnisse der Zwischensequenz (SP 11, As 24-53, Tab. 6) deutlich, daß in dem folgenden Sequenzbereich (AS 39 - 47) keine immunrelevanten Epitope vorhanden sind und die sehr schwache, noch nachzuweisende Reaktivität des SP 11 durch den darin enthaltenen Carboxy-terminalen Bereich des SP 10 zurückzuführen ist (überlappender Bereich AS 24-30 gemäß Fig. 3).

Tab. 6:

| Vergleich der Immunreaktivitäten im ELISA zwischen 2 erfindungsgemäßen Peptiden und einer Aminosäuresequenz, die zwischen der publizierten Sequenz (AS 39-74), OKAMOTO et al.) und den neuen Strukturen liegt (SP 11, AS 24 -53, siehe auch Fig. 3). Alle Angaben stellen Extinktionswerte dar ($E_{450nm}$). | | | |
|---|---|---|---|
| Proben | SP 23 AS 8-26 2 µg/ml | SP 10 AS 1-30 2 µg/ml | SP 11 AS 24-53 2 µg/ml |
| pos. Kontr. | > 2.500 | > 2.500 | 0.405 |
| neg. Kontr. | 0.051 | 0.049 | 0.030 |
| Verdünnungen | | | |
| HC-90-225 | | | |
| 1 : 16 | 2.203 | > 2.500 | 0.428 |
| 1 : 64 | 1.329 | 1.367 | 0.164 |
| 1 : 256 | 0.454 | 0.386 | 0.024 |
| 1 : 1024 | 0.195 | 0.142 | |
| HC-90-354 | | | |
| 1 : 16 | > 2.500 | > 2.500 | 0.164 |
| 1 : 64 | 1.413 | 2.156 | 0.059 |
| 1 : 256 | 0.418 | 0.496 | |
| 1 : 1024 | 0.124 | 0.162 | |
| Anti HCV-positive Proben (kommerzieller Test HP 1) | | | |
| HC 90-371 | 2.200 | 2.086 | 0.224 |
| HC 90-336 | > 2.500 | > 2.500 | 0.335 |
| HC 90-453 | > 2.500 | > 2.500 | 0.058 |
| HC 90-570 | 1.877 | 2.277 | 0.088 |

Besonders deutlich wird dieser überraschende Empfindlichkeitsvorteil bei der Testung von unverdünnten Proben. So werden von den in Tab. 7 wiedergegebenen 11 Anti HCV-positiven Proben mit dem erfindungsgemäßen Peptid alle 11 als reaktiv ermittelt, während das Literaturbeschriebene Peptid nur 6 Proben positiv findet und beim kommerziellen Test (HP1) in keinem Fall positiv reagiert. In Bezug auf die mit den beiden Peptiden übereinstimmend positiv reagierenden Proben fällt auf, daß das erfindungsgemäße Peptid unter gleichen Testbedingungen signifikant stärker reagiert, was insbesondere für die Positiv/Negativ-Diskriminierung erhebliche Vorteile bietet.

EP 0 484 787 B1

Tab. 7

Vergleich der Sensitivität eines Peptid-ELISAs (SP10 2 μg/ml) zum Stand der Technik. Dazu wurden 11 ausgewählte native humane Anti HCV-positive Proben mit einem ELISA basierend auf einem erfindungsgemäßen Peptid vergleichend getestet mit einem kommerziellen Test (HP1) sowie einem Peptid, das gemäß der Fig. 3 in der Literatur als immunrelevant beschrieben wird (OKAMOTO et al.). Alle Angaben stellen Extinktionswerte dar ($E_{450\ nm}$). Die Anti-HCV positive Probe HC 90-495 wurde als Kontrolle mitgetestet.

| Serum No. | erfindungsgemäßes Peptid SP10 2 μg/ml cut off: 0,100 E | literaturbeschriebenes Peptid (OKAMOTO) SP12 2 μg/ml cut off: 0,100 E | | kommerzieller HCV-ELISA (HP1) cut off 0,454 E | |
|---|---|---|---|---|---|
| HC 90-224 | 1,952 | 2,005 | | 0,114 | Neg. |
| HC 90-284 | 2,417 | 0,451 | | 0,095 | Neg. |
| HC 90-289 | > 2,500 | 1,220 | | 0,278 | Neg. |
| HC 90-300 | > 2,500 | 0,288 | g.w. | 0,208 | Neg. |
| HC 90-323 | > 2,500 | > 2,500 | | 0,313 | Neg. |
| HC 90-493 | > 2,500 | 0,051 | Neg. | 0,088 | Neg. |
| HC 90-509 | > 2,500 | 0,081 | Neg. | 0,083 | Neg. |
| HC 90-512 | > 2,500 | 0,049 | Neg. | 0,110 | Neg. |
| HC 90-531 | 2,208 | 0,257 | g.w. | 0,081 | Neg. |
| HC 90-536 | 2,500 | 0,234 | g.w. | 0,069 | Neg. |
| HC 90-494 | 1,279 | n.d. | | 0,114 | Neg. |
| HC 90-495 | > 2,500 | > 2,500 | | > 2,500 | Pos. |

Diese anhand der Titrationen (Tab. 5) und vorselektierten nativen Humanseren abgeleiteten Befunde (Tab. 7) mit den core-Peptiden werden bestätigt durch die Resultate der Testung von kommerziell erhältlichen Serumpanel (LOT # PHV 101 und LOT # PHV 201, Boston Biomedica, USA).

Im Einzelnen sind die Ergebnisse des sog. low titre Anti HCV-Panel in Tab. 8 und die Resultate des Mixed titre Anti HCV-Panel in Tab. 9 zusammengefaßt. Den Ergebnissen der mit dem erfindungsgemäßen core-Peptid erhobenen Daten wurden die Daten, die mit kommerziellen Tests als Stand der Technik erhoben wurden, gegenübergestellt

<u>**Tab. 8**</u>

**Die Tab. 8 zeigt den Vergleich der Sensitivität eines Peptid-ELISAs (SP 10, 2 µg/ml) zum Stand der Technik bei nativen Anti HCV-positiven Proben des Panels PHV 101, umfassend 15 niedrigtitrige gut charakterisierte Anti HCV-positive Proben (Vertrieb des Materials und der Vergleichs-Testergebnisse durch Fa. Boston Biomedica, USA). Alle ELISA-Angaben erfolgen als ratio-Werte, womit das Verhältnis von Proben-Extinktion zu cut-off beschrieben wird. Werte von < 1,0 werden als negativ und > 1,0 als positiv angesehen.**

Tab. 8

| Member I.D. Number | Vergleichsergebnisse | | | ELISA mit erfin- dungsgem. Peptid (SP10/2µg/ml) |
|---|---|---|---|---|
| | HCV EIA S/CO | HCV ELISA S/CO | RESULT | S/CO |
| PHV101-01 | 3.717 | 0.964 | POS | 18 + |
| PHV101-02 | 0.167 | 0.268 | N | 0,25 − |
| PHV101-03 | 1.880 | 1.323 | POS | 0,90 − |
| PHV101-04 | 1.757 | 3.076 | POS | > 25 |
| PHV101-05 | 3.404 | 2.169 | POS | 4,0 |
| PHV101-06 | 1.640 | 1.642 | POS | > 25 |
| PHV101-07 | 2.281 | 1.624 | POS | > 25 |
| PHV101-08 | 3.009 | 1.588 | POS | > 25 |
| PHV101-09 | 1.855 | 1.119 | POS | > 25 |
| PHV101-10 | 3.649 | 3.098 | POS | > 25 |
| PHV101-11 | 0.662 | 1.820 | POS | > 25 |
| PHV101-12 | 3.018 | 2.205 | POS | > 25 |
| PHV101-13 | 2.805 | 2.770 | POS | > 25 |
| PHV101-14 | 1.912 | 0.846 | POS | > 25 |
| PHV101-15 | 3.686 | 3.240 | POS | > 25 |

EP 0 484 787 B1

Fortsetzung Tab. 8

| MEMBER I.D. Number | CONFIRMATORY DATA RIBA 2.0 | | | | RESULT* | ALT** | anti-HBc* | HBsAg* | anti-HIV* | anti-HTLV* | ELISA mit erfindungsgem. Peptid (SP 10/ 2μg/ml) S/Co |
|---|---|---|---|---|---|---|---|---|---|---|---|
| | 5-1-1 | C-100-3 | C33C | C22-C | | | | | | | |
| PHV101-01 | 1 | +/- | +/- | 2 | POS | L | N | N | N | N | 18 + |
| PHV101-02 | - | - | - | - | N | L | N | N | N | N | 0,25 - |
| PHV101-03 | +/- | +/- | +/- | - | N | L | N | N | N | N | 0,90 - |
| PHV101-04 | +/- | +/- | 4 | 4 | POS | L | POS | N | N | N | > 25 |
| PHV101-05 | - | +/- | - | +/- | N | L | N | N | N | N | 4,0 |
| PHV101-06 | +/- | - | 1 | 4 | POS | L | POS | N | N | N | > 25 |
| PHV101-07 | - | +/- | 1 | 4 | POS | L | POS | N | N | N | > 25 |
| PHV101-08 | +/- | - | 1 | 4 | POS | G | POS | N | N | N | > 25 |
| PHV101-09 | - | - | 1 | 4 | POS | L | N | N | N | N | > 25 |
| PHV101-10 | 1 | - | 2 | 4 | POS | L | POS | N | N | N | > 25 |
| PHV101-11 | 1 | +/- | 1 | 1 | POS | L | N | N | N | N | > 25 |
| PHV101-12 | 2 | - | 4 | 4 | POS | L | POS | N | N | N | > 25 |
| PHV101-13 | +/- | +/- | 4 | 3 | POS | L | POS | N | N | N | > 25 |
| PHV101-14 | +/- | - | 1 | 3 | POS | L | POS | N | N | N | > 25 |
| PHV101-15 | 1 | - | 3 | 4 | POS | L | POS | N | N | N | > 25 |

*POS = Positive, N = Negative, NA = Not Applicable
**L = Less than 2 x upper limit of normal, G = Greater than 2 x upper limit of normal.
ALT results are those read following sterile filtration and vialing and may not reflect the ALT status of the donor at the time of donation.

EP 0 484 787 B1

Aus den Daten der Tab. 8 wird ersichtlich, daß mit einer Ausnahme (PHV 01-03) alle Anti-HCV-positiven Proben richtig positiv mit dem neuen Peptid reagieren. Im Vergleich mit den kommerziellen Tests fällt auf, daß die Signalstärke, ausgedrückt im ratio Signal der Probe: cut off mit dem erfindungsgemäßen Peptid signifikant stärker ermittelt wurde als mit den vergleichend geprüften Assays, worin sich eine deutlich bessere Zuverlässigkeit des neuen Peptid-ELISAs ausdrückt.

Gemäß diesen Daten (ratio > 25, was Extinktionen von > 2,5 entspricht), handelt es sich nur nach der Definition gemäß des Standes der Technik um niedrig-titrige Proben, die mit dem neuen Peptid überraschend als stark reaktiv ermittelt werden.

Die eine Ausnahme (Probe 03) erklärt sich aus den Daten des Vergleichsergebnisses aus dem Bestätigungstest, der ein Fehlen von core-spezifischen Antikörpern in dieser Probe nachweist (C22c stellt das gentechnologisch in E. coli hergestellte core-Protein dar). Gemäß diesem Test wäre die Probe 03 sogar als negativ zu klassifizieren.

In ähnlichen Beobachtungen führt die Testung des zweiten in Tab. 9 zusammengefaßten Panels: von den ingesamt 22 Anti HCV-positiven Proben werden 19 mit Extinktionen von größer 2,5 (entspricht ratio > 25) überraschend extrem stark positiv gefunden. Die drei in dem Panel enthaltenen Anti HCV-negativen Seren werden richtig als negativ klassifiziert (ratios < 1,0). Schließlich werden auch die Proben Nr. PHV 201-08, -10 und -20 im Sinne der Fragestellung richtig gefunden, da diese Proben keine (-08 und -10) bzw. wenig (-20) core-spezifische Antikörper gemäß dem Bestätigungstest aufweisen.

Tab. 9 zeigt den Vergleich der Empfindlichkeit eines Peptid-ELISAs (SP 10, 2 µg/ml) zum Stand der Technik bei nativen Anti HCV-positiven Proben des Panel PHV 201, umfassend gut charakterisierte Anti HCV-positive Proben mit unterschiedlichen Anti HCV-Titern. (Vertrieb des Materials und der Vergleichs-Testergebnisse durch die Firma Boston Biomedica, USA). Alle ELISA Angaben werden als ratios wiedergegeben, die den Quotienten aus Proben-Extinktion und cut-off-Wert darstellen. Werte von < 1,0 stellen ein Negativ- und Werte von > 1,0 ein Positiv-Ergebnis dar.

EP 0 484 787 B1

Tab. 9    Vergleichsergebnisse

| MEMBER I.D. NUMBER | HCV EIA | HCV ELISA | RESULT | ELISA mit erfindungsgem. Peptid (SP 10/ 2 µg/ml) S/Co |
|---|---|---|---|---|
| PHV201-01 | 1.689 | 1.475 | POS | > 25 |
| PHV201-02 | 3.668 | 5.660 | POS | > 25 |
| PHV201-03 | 3.793 | 3.946 | POS | > 25 |
| PHV201-04 | 0.271 | 0.176 | N | 0,10 - |
| PHV201-05 | 3.417 | 2.591 | POS | > 25 |
| PHV201-06 | 3.711 | 4.805 | POS | > 25 |
| PHV201-07 | 0.260 | 0.108 | N | 0,25 - |
| PHV201-08 | 1.515 | 1.244 | POS | 0,75 - |
| PHV201-09 | 2.530 | 1.274 | POS | > 25 |
| PHV201-10 | 2.174 | 2.745 | POS | 0,25 - |
| PHV201-11 | 2.883 | 2.169 | POS | > 25 |
| PHV201-12 | 3.641 | 4.769 | POS | > 25 |
| PHV201-13 | 4.115 | 5.662 | POS | > 25 |
| PHV201-14 | 2.669 | 4.300 | POS | > 25 |
| PHV201-15 | 4.115 | 5.660 | POS | > 25 |
| PHV201-16 | 4.115 | 5.662 | POS | > 25 |
| PHV201-17 | 4.115 | 5.662 | POS | > 25 |
| PHV201-18 | 3.530 | 3.943 | POS | > 25 |
| PHV201-19 | 0.498 | 1.520 | N | 0,25 - |
| PHV201-20 | 2.611 | 2.377 | POS | > 25 |
| PHV201-21 | 3.430 | 2.666 | POS | > 25 |
| PHV201-22 | 1.721 | 1.690 | POS | > 25 |
| PHV201-23 | 4.115 | 5.662 | POS | > 25 |
| PHV201-24 | 2.500 | 2.631 | POS | > 25 |
| PHV201-25 | 4.115 | 5.660 | POS | > 25 |

EP 0 484 787 B1

Fortsetzung Tab. 9

| MEMBER I.D. NUMBER | CONFIRMATORY DATA RIBA 2.0 | | | | RESULT* | ALT** | anti-HBc* | HBsAg* | anti-HIV* | anti-HTLV* | ELISA mit erfin-dungsgem. Peptid (SP10 / 2 µg/ml) S/Co |
|---|---|---|---|---|---|---|---|---|---|---|---|
| | 5-1-1 | C-100-3 | C33C | C22-C | | | | | | | |
| PHV201-01 | +/- | – | 2 | 4 | POS | L | N | N | N | N | > 25 |
| PHV201-02 | 1 | 2 | 4 | 4 | POS | L | POS | N | N | N | > 25 |
| PHV201-03 | – | 1 | – | 4 | POS | L | N | N | N | N | > 25 |
| PHV201-04 | – | – | – | – | N | L | N | N | N | N | 0,10 – |
| PHV201-05 | 2 | +/- | +/- | 4 | POS | L | N | N | N | N | > 25 |
| PHV201-06 | 2 | 1 | 4 | 4 | POS | L | N | N | N | N | > 25 |
| PHV201-07 | – | – | – | – | N | L | N | N | N | N | 0,25 – |
| PHV201-08 | – | +/- | 3 | – | I | L | POS | N | N | N | 0,75 – |
| PHV201-09 | 1 | – | 4 | 4 | POS | L | POS | N | N | N | > 25 |
| PHV201-10 | +/- | +/- | 2 | – | I | G | N | N | N | N | 0,25 – |
| PHV201-11 | 2 | +/- | 3 | 3 | POS | G | N | N | N | N | > 25 |
| PHV201-12 | 2 | 2 | 2 | 3 | POS | L | N | N | N | N | > 25 |
| PHV201-13 | 4 | 4 | 4 | 4 | POS | L | | N | N | N | > 25 |
| PHV201-14 | 2 | +/- | 4 | 4 | POS | L | POS | N | N | POS | > 25 |
| PHV201-15 | 2 | +/- | 4 | 4 | POS | L | N | N | N | N | > 25 |
| PHV201-16 | 4 | 4 | 4 | 4 | POS | L | N | N | N | N | > 25 |
| PHV201-17 | 4 | 4 | 4 | 4 | POS | L | POS | N | N | N | > 25 |
| PHV201-18 | +/- | 2 | 4 | 3 | POS | L | POS | N | N | N | > 25 |
| PHV201-19 | – | +/- | – | – | N | L | N | N | N | N | 0,25 – |
| PHV201-20 | 1 | 1 | 1 | +/- | POS | L | N | N | N | N | > 25 |
| PHV201-21 | 1 | +/- | 3 | 4 | POS | L | POS | N | N | N | > 25 |
| PHV201-22 | +/- | – | 4 | 4 | POS | L | N | N | N | N | > 25 |
| PHV201-23 | 4 | 3 | 4 | 4 | POS | L | POS | N | N | N | > 25 |
| PHV201-24 | 2 | 4 | 4 | 4 | POS | G | POS | POS | N | N | > 25 |
| PHV201-25 | 4 | 1 | 4 | 4 | POS | L | N | N | N | N | > 25 |

*POS = Positive, N = Negative, I = Indeterminant, NA = Not Applicable

**L = Less than 2 x upper limit of normal, G = Greater than 2 x upper limit of normal.

ALT results are those read following sterile filtration and vialing and may not reflect the ALT status of the donor at the time of donation.

**Beispiel 6**

**Bestimmung von Anti HCV in Schimpansen-Seren mit den erfindungsgemäßen Peptiden im ELISA**

Verschiedene Peptide bzw. Peptid-Mischungen, adsorbiert in Vertiefungen von Mikrotiterplatten wurden mit Seren von Schimpansen untersucht, die mit verschiedenen infektiösen Dosen des NANBV infiziert wurden. Von den sequentiellen Entnahmen alle 2 Wochen post Inokulation wurden neben der GOT auch die GPT mit kommerziellen Tests gemessen und alle Proben parallel sowie im ELISA mit erfindungsgemäßen Peptiden bestimmt. Ähnlich wie beim kommerziellen Test (HP1) des Beispiels 3. wurden beim Peptid-ELISA die polyklonalen Kaninchen-Antikörper, markiert mit POD, 4fach stärker konzentriert zur Detektion verwendet, wobei das Enzymsubstrat TMB verwendet wurde mit photometrischer Messung bei 450 nm. Die Testdurchführung entsprach der vorstehend beschriebenen Bestimmung von humanen Anti HCV mit 0,1 E als Grenzwert-festlegung sowie Wiedergabe der Ergebnisse der Tab. 10 als Verhältnis der spezifischen Extinktionswerte zu diesem cut off-Wert (ratios).

Die in Tab. 10A bis 10C und 11A und 11B aufgeführten Ergebnisse mit Peptiden aus dem NSP 4-Bereich stellen sogenannte "ratios" dar, mit denen der Quotient aus spezifischem Signal und Grenzwert definiert wird.

**Tab. 10 A - C**
**Anti HCV-Bestimmung in Schimpansen-Proben mit einem kom-merziellen Test (HP1) und ELISAs mit erfindungsgemäßen Peptiden bzw. Peptidmischungen aus dem NSP 4-Bereich. Die Angaben erfolgen bei den ersten 3 Zeitwerten in Extink-tionen, die für die Festlegung der Grenzwerte dienen, woraus die nachfolgenden ratios aus spezifischem Signal und Grenzwert gebildet wurden.**

## Tab. 10 A

| Tier Nr. 123* Zeit (Wochen) | ALT | kommerz. Test (HP1) | 4083 2 $\mu$g/ml | 4083 0,5 $\mu$g/ml | 4060 4081 je 2 $\mu$g/ml |
|---|---|---|---|---|---|
| 0 = Inokulation | 25 | 0,500 E$_{492}$ | 0,032 E$_{450}$ | 0,041 E$_{450}$ | 0,026 E$_{450}$ |
| 2 | 23 | 0,600 E | 0,027 E | 0,024 E | 0,014 E |
| 4 | 23 | 0,450 E | 0,013 E | 0,025 E | 0,019 E |
| Extinktionswerte (E) definieren die Grenzwerte: | | 0,7 E | 0,10 E | 0,10 E | 0,10 E |
| ratios (r): | | | | | |
| 6 | 29 | r 0,6 | r 0,01 | r 0,03 | r 0,03 |
| 8 | 30 | r 0,4 | r 0,02 | r 0,03 | r 0,03 |
| 10 | 50 | r 0,7 | r 0,01 | r 0,02 | r 0,02 |
| 12 | 55 | r 0,4 | r 0,02 | r 0,03 | r 0,03 |
| 14 | 50 | r 0,5 | r 0,01 | r 0,03 | r 0,03 |
| 16 | 140 | r 0,6 | r 0,03 | r 0,03 | r 0,03 |
| 18 | > 170 | r 0,7 | r 5,3 pos. | r 1,5 pos. | r 0,6 |
| 20 | 30 | r 0,6 | r 7,4 pos. | r 1,8 pos. | r 0,7 |
| 22 | 23 | r 0,5 | r 5,7 pos. | r 1,1 pos. | r 0,5 |
| 24 | 23 | r 0,6 | r 2,7 pos. | r 0,7 | r 0,3 |
| 26 | 30 | r 0,6 | r 1,5 pos. | r 0,4 | r 0,3 |

*) durch elektronenmikroskopische Untersuchung von Leberbiopsien
als NANB-infiziert gesichert

EP 0 484 787 B1

**Tab. 10 B**

| Tier Nr. 048 Zeit (Wochen) | ALT | kommerz. Test (HP1) | 4083 2 µg/ml | 4083 0,5 µg/ml | 4060 4081 je 2 µg/ml | 4073 2 µg/ml | 4090 2 µg/ml |
|---|---|---|---|---|---|---|---|
| 0 = Inokulation | 20 | 0,5 $E_{492}$ | 0,018 $E_{450}$ | 0,027 $E_{450}$ | 0,008 $E_{450}$ | 0,036 $E_{450}$ | 0,028 $E_{450}$ |
| 2 | 25 | 0,5 $E_{492}$ | 0,041 $E_{450}$ | 0,070 $E_{450}$ | 0,007 $E_{450}$ | 0,041 $E_{450}$ | 0,050 $E_{450}$ |
| 4 | 23 | 0,5 E | 0,026 E | 0,050 E | 0,009 E | 0,039 E | 0,037 E |
| Extinktionswerte (E) definieren die Grenzwerte: | | 0,70 E | 0,10 E | 0,10 E | 0,10 E | 0,10 E | 0,10 E |
| ratios (r) | | | | | | | |
| 10 | 32 | r 0,6 | r 0,3 | r 0,5 | r 0,2 | r 0,6 | r 0,6 |
| 12 | 100 | r 0,4 | r 1,0 gw[1] | r 1,0 gw[1] | r 0,1 | r 0,95 gw[1] | r 2,0 pos. |
| 14 | 112 | r 0,7 | r11,7 pos. | r 4,9 pos. | r 0,1 | r 6,9 pos. | r16,2 pos. |
| 16 | 30 | r 1,0 | r 7,4 pos. | r 3,8 pos. | r 0,2 | r12,2 pos. | r23,2 pos. |
| 18 | 25 | r 0,7 | r 7,2 pos. | r 2,8 pos. | r 0,2 | r 7,8 pos. | r18,9 pos. |
| 20 | 30 | r 0,8 | r 8,6 pos. | r 4,7 pos. | r 0,1 | r12,8 pos. | r16,4 pos. |
| 22 | 25 | r 0,8 | r 8,4 pos. | r 5,4 pos. | r 0,1 | r11,7 pos. | r22,3 pos. |
| 24 | 23 | r 0,8 | r 9,8 pos. | r 7,2 pos. | r 0,2 | r15,5 pos. | r20,0 pos. |
| 26 | 20 | r 0,8 | r10,8 pos. | r 6,0 pos. | r 0,2 | r13,0 pos. | r17,4 pos. |
| 28 | 20 | r 0,8 | r 9,8 pos. | r 5,4 pos. | r 0,2 | r11,7 pos. | r 8,9 pos. |
| 30 | 20 | r 1,0 gw[1] | r 7,6 pos. | r 6,3 pos. | r 0,2 | r11,5 pos. | r12,9 pos. |
| 32 | 25 | r 1,3 pos. | r16,2 pos. | r17,4 pos. | r 1,3 pos. | r24,5 pos. | r23,3 pos. |
| 34 | 25 | r 2,0 pos. | r18,4 pos. | r22,5 pos. | r 2,0 pos. | r >25 pos. | r >25 pos. |
| 36 | 28 | r 2,4 pos. | r16,1 pos. | r22,0 pos. | r 2,0 pos. | r >25 pos. | r >25 pos. |
| 38 | 28 | r 2,1 pos. | r17,9 pos. | r >25 pos. | r 2,1 pos. | r >25 pos. | r >25 pos. |
| 40 | 26 | r 2,9 pos. | r >25 pos. | r >25 pos. | r 2,5 pos. | r >25 pos. | r >25 pos. |

[1] gw = grenzwertig positiv

Tab. 10 C

| Tier Nr. 147 Zeit (Wochen) | ALT | kommerz. Test (HP1) | 4083 2 $\mu$g/ml | 4083 0,5 $\mu$g/ml | 4060 4081 je 2 $\mu$g/ml | 4074 4081 je 2 $\mu$g/ml |
|---|---|---|---|---|---|---|
| 0 = Inokulation | 30 | 0,60 $E_{492}$ | 0,068 $E_{450}$ | 0,042 $E_{450}$ | 0,008 $E_{450}$ | 0,027 $E_{450}$ |
| 2 | 25 | 0,50 $E$ | 0,043 $E$ | 0,029 $E$ | 0,012 $E$ | 0,032 $E$ |
| 4 | 30 | 0,45 E | 0,066 E | 0,033 E | 0,015 E | 0,023 E |
| Extinktionswerte (E) definieren die Grenzwerte | | 0,70 E | 0,10 E | 0,10 E | 0,10 E | 0,10 E |
| ratios (r): | | | | | | |
| 16 | 52 | r 0,5 | r 0,2 | r 0,2 | r 0,1 | r 0,3 |
| 18 | 75 | r 0,5 | r 1,1 pos. | r 0,3 | r 0,1 | r 0,2 |
| 20 | 110 | r 0,8 | r 6,5 pos. | r 1,6 pos. | r 0,2 | r 0,3 |
| 22 | 40 | r 0,6 | r11,8 pos. | r 5,9 pos. | r 0,2 | r 0,5 |
| 24 | 75 | r 0,6 | r 7,6 pos. | r 3,7 pos. | r 0,1 | r 1,0 gw[1] |
| 26 | 70 | r 0,4 | r12,5 pos. | r 6,0 pos. | r 0,1 | r 2,2 pos. |
| 28 | 55 | r 0,5 | r10,9 pos. | r 4,9 pos. | r 0,2 | r 2,1 pos. |
| 30 | 52 | r 0,6 | r 8,9 pos. | r 6,2 pos. | r 0,4 | r 3,5 pos. |
| 32 | 42 | r 0,7 | r11,3 pos. | r 6,9 pos. | r 0,9 | r 3,4 pos. |
| 34 | 48 | r 0,8 | r13,3 pos. | r14,1 pos. | r 1,6 pos. | r 7,6 pos. |
| 36 | 48 | r 0,8 | r19,9 pos. | r19,7 pos. | r 2,8 pos. | r 9,5 pos. |
| 38 | 35 | r 1,3 pos. | r20,8 pos. | r24,7 pos. | r 2,5 pos. | r11,0 pos. |
| 40 | 45 | r 1,3 pos. | r >25 pos. | r >25 pos. | r 3,4 pos. | r15,0 pos. |

1) gw = grenzwertig positiv

EP 0 484 787 B1

Die in den Tab. 10 A - C dargestellten Ergebnisse unterstreichen die Vorteilhaftigkeit der erfindungsgemäßen Peptide, insbesondere wenn man mit den entsprechenden Resultaten des kommerziellen Tests (HP1) und dem ALT-Verlauf vergleicht.

So wird beispielsweise Tier Nr. 123 vom kommerziellen Test (HP1) gar nicht Antikörper-positiv gefunden, obwohl anhand von Leberbiopsien elektronenmikroskopisch eine NANBH nachgewiesen wurde. Demgegenüber wird mit dem ELISA basierend auf 4083 fast gleichzeitig mit dem ALT-Anstieg ein zuverlässiger HCV-Antikörper-Nachweis möglich.

Insbesondere die zeitliche Kongruenz der Peptid-ELISA-Ergebnisse mit den angestiegenen ALT-Werten wird bei Tier Nr. 048 deutlich, wo mit allen beschriebenen Peptiden bzw. Peptid-Mischungen ein Anti-HCV-Nachweis fast gleichzeitig mit dem ALT-Anstieg und durchschnittlich 18 Wochen vor dem kommerziellen Test (HP1) im Sinne einer scharfen Positiv-Negativ-Diskriminierung sehr zuverlässig erfolgt. Insbesondere die Vergleiche unter den Peptiden legen nahe, daß die Erkennung der frühen HCV-Antikörper durch das Gesamtpeptid der Formel I (4083) erheblich durch die mittleren Aminosäuresequenz mitbestimmt wird wie der Vergleich der Mischung aus 4060 und 4081 (Amino- und Carboxy-terminale Sequenzen) und dem die mittlere Struktur umfassenden 4090 deutlich macht.

Zu ähnlichen Ergebnissen einer frühen, fast mit ALT zeitgleichen Erfassung von HCV-Antikörpern führten die Untersuchungen des Tieres Nr. 147, bei dem ein zuverlässiger HCV-Antikörper-Nachweis etwa zeitgleich mit dem ALT-Anstieg wiederum ca. 16 - 18 Wochen früher möglich ist als mit dem kommerziellen Test (HP1).

**Tab. 11 A u. B**
**Anti HCV-Bestimmung in Serum-Proben von 2 Schimpansen, die mit HCV infiziert wurden. Dargestellt werden die Ergebnisse mit dem kommerziellen Test (HP1) im Vergleich zu einem erfindungsgemäßen synthetisch hergestellten core-Peptid (SP 10, 2 μg/ml), wobei die Ergebnisse als ratios aus spezifischem Signal und Grenzwert dargestellt werden.**

Tier Nr. 048

| Zeit (Wochen) | ALT | kommerzieller HCV ELISA (HP1) | SP 10-ELISA 2 µg/ml |
|---|---|---|---|
| O = Inokulation | 20 | $0,5\ E_{492}$ | $0,09\ E_{450}$ |
| 2 | 25 | 0,5 E | 0,21 E |
| 4 | 23 | 0,5 E | 0,13 E |

Extinktionswerte
(E) definieren die
Grenzwerte:

| | | 0,70 E | 0,10 E |
|---|---|---|---|
| 8 ratios (r) | 34 | r 0,4 | 0,50 |
| 10 | 32 | r 0,6 | r >20,0 pos. |
| 12 | 100 | r 0,4 | r >20,0 pos. |
| 14 | 112 | r 0,7 | r >20,0 pos. |
| 16 | 30 | r 1,0 | r >20,0 pos. |
| 18 | 25 | r 0,7 | r >20,0 pos. |
| 20 | 30 | r 0,8 | r >20,0 pos. |
| 22 | 25 | r 0,8 | r 19,0 pos. |
| 24 | 23 | r 0,8 | r 20,0 pos. |
| 26 | 20 | r 0,8 | r 18,5 pos. |
| 28 | 20 | r 0,8 | r 10,4 pos. |
| 30 | 20 | r 1,0 gw[1] | r 14,2 pos. |
| 32 | 25 | r 1,3 pos. | r 17,6 pos. |
| 34 | 25 | r 2,0 pos. | r 15,4 pos. |
| 36 | 28 | r 2,4 pos. | r >20,0 pos. |
| 38 | 28 | r 2,1 pos. | r >20,0 pos. |
| 40 | 26 | r 2,9 pos. | r >20,0 pos. |

1) gw = grenzwertig positiv

Tier Nr. 147

| Zeit (Wochen) | ALT | kommerzieller HCV ELISA (HP1) | SP 10-ELISA 2µg/ml |
|---|---|---|---|
| O = Inokulation | 30 | $0,60\ E_{492}$ | $0,011\ E_{450}$ |
| 2 | 25 | 0,50 E | 0,032 |
| 4 | 30 | 0,45 E | 0,029 |

Extinktionswerte
(E) definieren die
Grenzwerte:

|  |  |  | 0,70 E | 0,10 E |
|---|---|---|---|---|
| 16 ratios (r) | 52 | r 0,5 | r | 0,2 |
| 18 | 75 | r 0,5 | r | 0,1 |
| 20 | 110 | r 0,8 | r | 0,2 |
| 22 | 40 | r 0,6 | r | 0,4 |
| 24 | 75 | r 0,6 | r | 0,3 |
| 26 | 70 | r 0,4 | r | 0,2 |
| 28 | 55 | r 0,5 | r | 0,3 |
| 30 | 52 | r 0,6 | r | 0,2 |
| 32 | 42 | r 0,7 | r | 0,4 |
| 34 | 48 | r 0,8 | r >20,0 pos. | |
| 36 | 48 | r 0,8 | nicht getestet | |
| 38 | 35 | r 1,3 pos. | nicht getestet | |
| 40 | 45 | r 1,3 pos. | r >20,0 pos. | |

1) gw = grenzwertig positiv

Auch die in den Tab. 11 A und B dargestellten Ergebnisse mit einem neuen core-Peptid unterstreichen die Vorteilhaftigkeit der erfindungsgemäßen Peptide, insbesondere wenn man mit den entsprechenden Resultaten des kommerziellen ELISAs (HP1) und dem ALT-Verlauf vergleicht.

Insbesondere die zeitliche Kongruenz der core-Peptid-ELISA-Ergebnisse mit den angestiegenen ALT-Werten wird bei Tier Nr. 048 deutlich, wo ein Anti-HCV-Nachweis fast gleichzeitig mit dem ALT-Anstieg und durchschnittlich 20 Wochen vor dem kommerziellen ELISA (HP1) im Sinne einer scharfen positiv-negativ-Diskriminierung sehr zuverlässig erfolgt.

Zu ähnlichen Ergebnissen einer frühen, fast mit ALT zeitgleichen Erfassung von HCV-Antikörpern führten die Untersuchungen des Tieres Nr. 147, bei dem ein zuverlässiger HCV-Antikörper-Nachweis wiederum ca. 4 Wochen früher möglich ist als mit dem kommerziellen Test (HP1).

Insgesamt stellen sich die erfindungsgemäßen Peptide bzw. deren Verwendung in immunchemischen Nachweisverfahren wesentlich sensitiver dar, als alle bisher beschriebenen Verfahren basierend auf gentechnologisch hergestellten Proteinen sowie anderen synthetischen Peptiden des Standes der Technik. Bei verbesserter Sensitivität in späteren Infektionsphasen bieten die neuen Peptide zusätzlich wesentliche Vorteile, indem die Bestimmung früher HCV-Antikörper zuverlässig möglich wird und damit die z. Z. bestehende diagnostische Lücke signifikant verkleinert wird. Darüber hinaus stellen sich die erfindungsgemäßen Peptide wesentlich unempfindlicher gegenüber unspezifischen Bindungen dar, was sich nicht zuletzt in einem gegenüber dem kommerziellen Test (HP1) drastisch verringerten background ausdrückt (max. 0,10 $E_{450}$) gegenüber max. 0,4 $E_{492}$ beim kommerziellen Test (HP1), und dies sowohl bei Proben tierischer als auch menschlicher Herkunft, so daß eine wesentlich schärfere Negativ-Positiv-Diskriminierung möglich wird. Schließlich sind als vorteilhafte Aspekte die kürzere Gesamtdauer und präzisere Bestimmung durch das genauer zu pipettierende Probenvolumen von 50 µl zu nennen.

Beispiel 7:

**Herstellung von Peptid-Lösungen zur Erzeugung einer Mischung aus NSP 4-Peptid 4083 und core-Peptid SP 10 sowie Beschichtung von Mikrotitrationsplatten mit dieser Peptid-Mischung**

Aus Stammlösungen der Peptide SP 10 und 4083 in 50 % Essigsäure in destilliertem Wasser, enthaltend je 6 mg Peptid/ml wurden 2fache Verdünnungsreihen in 0,10 M Natriumbicarbonat pH 9,6 angelegt, also eine Reihe mit den Konzentrationen 50; 25; 12,5; 6,25; 3,12; 1,56; 0,78; 0,39; 0,2; 0,1; 0,05 und 0,01 µg Peptid/ml erhalten. Bei Mischungen von einzelnen Peptiden wurde analog vorgegangen, wobei die Stammlösungen zusätzlich in verschiedenen Verhältnissen gemischt wurden, z. B. 10 : 1 oder 1 : 4, um durch Verdünnung in 0,1 M Natriumbicarbonat die oben angegebenen Gesamt-Endkonzentrationen zu erhalten, die aber bei Mischungen mehrerer Peptide diese gleichkonzentriert (bei 1 : 1-Mischung) oder in verschiedenen Verhältnissen zueinander enthalten.

100 µl einer jeden Verdünnung wurde in jeweils 16 Vertiefungen von Mikrotiterplatten, Typ B der Firma Nunc, Roskilde, Dänemark gegeben. Die mit den Verdünnungen gefüllten Testplatten wurden 18 Stunden bei 20°C belassen, dann wurden die Lösungen in den Vertiefungen abgesaugt und die Vertiefungen 3 - 4 mal mit 300 µl einer Lösung von 10 g/l Rinderserumalbumin in phosphatgepufferter physiologischer Kochsalzlösung, (PBS, pH 7,4) durch Füllen und Absaugen gewaschen und die Testplatten anschließend über Kieselgel bei 20°C getrocknet.

Als geeignet hat sich eine Konzentration von 1 µ g 40 83/ml und 1 µg SP 10/ml für die Beschichtung der Peptid-Mischung herausgestellt, wobei diese Konzentration die Basis der in Tab. 12 bis 14 zusammengefaßten Ergebnisse darstellt, die wie in **Beispiel 4 und 5** beschrieben, erhalten wurden.

Im Gegensatz zu bisherigen Vergleichen zum Stand der Technik werden alle folgenden Gegenüberstellungen einer kommerziellen Anti-HCV-ELISA der 2. Generation (HP 2) wie in Beispiel 3 beschrieben, zum Gegenstand haben.

**Tab. 12 und 13:**

Die Angaben zu dem erfindungsgemäßen und kommerziellen ELISA HP 2 stellen sogenannte Endpunkt-Titer dar, mit denen die höchste Vorverdünnung eines Serums in Anti HCV-negativem Serum definiert wird, die in einem gegebenen Test reproduzierbar positiv ermittelt wurde.

Tab. 12:

| BBI Low titre Anti HCV PANEL PHV 101 | | |
|---|---|---|
| MEMBER I.D. NUMBER | erfindungsgemäßer ELISA SP 4083/SP 10 je 1 µg/ml | kommerzieller Anti HCV-Test (HP2) |
| PHV101-01 | 1 : 16 | 1 : 2 |
| PHV101-02 | negativ | negativ |
| PHV101-03 | 1 : 4 | 1 : 1 |
| PHV101-04 | 1 : 512 | 1 : 64 |
| PHV101-05 | 1 : 2 | 1 : 1 |
| PHV101-06 | 1 : 128 | 1 : 32 |
| PHV101-07 | 1 : 128 | 1 : 128 |
| PHV101-08 | 1 : 64 | 1 : 16 |
| PHV101-09 | 1 : 64 | 1 : 16 |
| PHV101-10 | 1 : 256 | 1 : 128 |

Tab. 12:   (fortgesetzt)

| BBI Low titre Anti HCV PANEL PHV 101 | | |
|---|---|---|
| MEMBER I.D. NUMBER | erfindungsgemäßer ELISA SP 4083/SP 10 je 1 µg/ml | kommerzieller Anti HCV-Test (HP2) |
| PHV101-11 | 1 : 32 | 1 : 16 |
| PHV101-12 | 1 : 512 | 1 : 128 |
| PHV101-13 | 1 : 16 | 1 : 64 |
| PHV101-14 | 1 : 64 | 1 : 32 |
| PHV101-15 | 1 : 128 | 1 : 64 |

Tab. 13:

| BBI Mixed titre Anti HCV PANEL PHV 201 | | |
|---|---|---|
| MEMBER I.D. NUMBER | erfindungsgemäßer ELISA SP 4083/SP 10 je 1 µg/ml | kommerzieller Anti HCV-Test (HP2) |
| PHV201-01 | 1 : 1000 | 1 : 128 |
| PHV201-02 | 1 : 1000 | 1 : 500 |
| PHV201-03 | 1 : 128 | 1 : 32 |
| PHV201-04 | negativ | negativ |
| PHV201-05 | 1 : 64 | 1 : 64 |
| PHV201-06 | 1 : 64 | 1 : 256 |
| PHV201-07 | negativ | negativ |
| PHV201-08 | 1 : 4 | 1 : 16 |
| PHV201-09 | 1 : 1000 | 1 : 512 |
| PHV201-10 | 1 : 4 | 1 : 16 |
| PHV201-11 | 1 : 512 | 1 : 128 |
| PHV201-12 | 1 : 32 | 1 : 16 |
| PHV201-13 | 1 : 256 | 1 : 256 |
| PHV201-14 | 1 : 64 | 1 : 64 |
| PHV201-15 | 1 : 512 | 1 : 1000 |
| PHV201-16 | 1 : 512 | 1 : 512 |
| PHV201-17 | 1 : 128 | 1 : 256 |
| PHV201-18 | 1 : 16 | 1 : 128 |
| PHV201-19 | negativ | negativ |
| PHV201-20 | 1 : 4 | 1 : 4 |
| PHV201-21 | 1 : 500 | 1 : 256 |
| PHV201-22 | 1 : 16 | 1 : 256 |
| PHV201-23 | 1 : 256 | 1 : 512 |
| PHV201-24 | 1 : 256 | 1 : 512 |
| PHV201-25 | 1 : 1000 | 1 : 512 |

Tab. 14:

| Ergebnisse der Testung von 930 gesunden Blutspendern, von denen gleichzeitig Serum und Plasma gewonnen wurde. | | |
|---|---|---|
|  | Spezifität in % nach initialer Testung | Spezifität in % nach Wiederholung |
| n = 930 Seren | 99,5 | 99,7 |
| n = 930 Plasmen | 99,6 | 99,7 |

Beispiel 8

**Herstellung von Peptid-Lösungen zur Erzeugung von Mischungen gemäß Formel XIV mit Peptiden der Formeln XVIII, XIX, XX und XXII sowie Beschichtung von Mikrotitrationsplatten mit diesen Peptid-Mischungen**

Die Polypeptide SPH 9 (Formel XVIII), SPH 20 (Formel XIX), 4083 (Formel XX) und SP 10 (Formel XXII) wurden in einer Konzentration von 6 mg/ml in 50 % (v/v) Essigsäure gelöst.

Diese 4 Stammlösungen wurden auf Volumenbasis in unterschiedlichen Verhältnissen wie in Beispiel 4 beschrieben gemischt und in 0,10 M Natriumbicarbonat (pH 9,6) so verdünnt, daß die Gesamtkonzentration der Polypeptide zwischen 0,2 und 8 μg/ml betrug.

Jeweils 100 μl der verdünnten Lösungen wurde in jeweils 16 Vertiefungen von Mikrotiterplatten, Typ B der Firma Nunc, Roskilde, Dänemark gegeben. Die gefüllten Testplatten wurden 18 Stunden bei 20° C inkubiert. Anschließend wurden die Lösungen abgesaugt und die Vertiefungen 3 - 4mal mit 300 μl einer Lösung von 10 g/l Rinderserumalbumin in phosphatgepufferter physiologischer Kochsalzlösung, (PBS, pH 7,4) gespült und die Testplatten anschließend über Kieselgel bei 20° C getrocknet.

Beispiel 9

**Optimierung der Konzentration der HIV 1-, HIV 2- und HCV-Peptide für die Beschichtungsmischung sowie Optimierung der ELISA-Bestimmung und Auswerte-Kriterien**

Beginnend mit einem Volumenverhältnis der in Beispiel 8 beschriebenen vier Stammlösungen von 1 : 1 : 1 : 1 (V/V) wurden alle vier veränderlichen Anteile unabhängig voneinander, aber unter Konstanthaltung der jeweils drei anderen variiert, um in der Beschichtungslösung folgende Endkonzentrationen der Einzelpeptide von HIV 1, HIV 2 und HCV zu erhalten (in μg/ml):

| Peptid XVIII | Peptid XIX | Peptid XX | Peptid XXII |
|---|---|---|---|
| 2 | 2 | 2 | 2 |
| 1 | 2 | 2 | 2 |
| 0,5 | 2 | 2 | 2 |
| 0,2 | 2 | 2 | 2 |
| 0,1 | 2 | 2 | 2 |
| 0,05 | 2 | 2 | 2 |

| Peptid XVIII | Peptid XIX | Peptid XX | Peptid XXII |
|---|---|---|---|
| 2 | 1 | 2 | 2 |
| 2 | 0,5 | 2 | 2 |
| 2 | 0,2 | 2 | 2 |
| 2 | 0,1 | 2 | 2 |
| 2 | 0,05 | 2 | 2 |
| 2 | 2 | 1 | 2 |
| ... | | | |
| usw. bis | | | |
| 0,05 | 0,05 | 0,05 | 0,05 |

Diese Mischungen wurden wie in Beispiel 8 beschrieben auf Mikrotitrationsplatten fixiert und wie in Beispiel 4 und 5 beschrieben im ELISA bewertet, wobei die Konzentration des Peroxidase-markierten Antikörpers gegen humanes Immunglobulin G ebenfalls optimiert wurde.

Die Proben, mit denen die Optimierung bewertet wurde, bestanden aus niedrigtitrigen Anti HIV 1-, Anti HIV 2-und Anti HCV-Proben, die durch serielle Verdünnungen von entsprechenden positiven Humanseren in negativem Humanserum hergestellt wurden. Daneben wurden mehrere Anti HIV und Anti HCV-negative Proben ebenfalls getestet, um die Hintergrundreaktion (d. h. die unspezifische Bindung bei einer gegebenen beschichteten Mikrotitrationsplatte) definieren zu können.

Als Auswahlkriterium wurde ein möglichst hohes spezifisches Signal, d. h. hohe Grenzempfindlichkeit in der Bestimmung der Titrationsreihen humaner Anti HIV- bzw. Anti HCV-positiver Proben bei gleichzeitigem niedrigem Hintergrund bei der Prüfung von Anti HIV- und Anti HCV-negativer Proben angelegt.

Nach diesen Kriterien wurden in der Regel günstige Beschichtungsmischungen gefunden, von denen folgende Mischung ausgewählt wurde:

| XXI | SPH | 9 | 0,500 µg/ml |
|-----|-----|------|-------------|
|     | SPH | 20 | 0,250 µ g/ml |
|     | SP | 4083 | 0,500 µg/ml |
|     | SP | 10 | 0,125 µg/ml |

Als günstig wurde eine Konjugat-Konzentration des Beispiels 2 von 1 : 3000 bei einer einheitlichen zusätzlichen Endverdünnung von 1 : 26 ermittelt, wobei die Testdurchführung nach Beispiel 4 erfolgte.

Im Gegensatz zur bisherigen Grenzwert-Festlegung wurden unter diesen Bedingungen Proben als Anti HIV- und/oder Anti HCV-positiv eingestuft, die eine Extinktion bei 450 nm aufwiesen, die größer ist als der Mittelwert der Negativ-Kontrollen zuzüglich eines Zuschlags von 0,250 O.D. (neue Grenzwert-Festlegung).

Diese Festlegungen wurden auf die folgenden Beispiele 10 bis 15 einheitlich und unverändert angewendet.

Beispiel 10

**Bestimmung von humanen Antikörpern der Immunglobulin-G-Klasse gegen HIV 1, HIV 2 und HCV im ELISA**

Die in Tab. 15 - 17 dargestellten Proben wurden wie im Beispiel 4 beschrieben unter optimierten Bedingungen des Beispiels 9 mit der Peptid-Mischung der Formel XIV getestet. Im Hinblick auf Anti HIV 1 oder 2 wurden die Reaktivitäten des erfindungsgemäßen Verfahrens verglichen mit einem Anti HIV 1/2-Kombinationstest (Enzygnost[R] Anti HIV 1/2, Fa. Behringwerke AG, HP 3). Zur Kontrolle wurden kommerziell erhältliche Western blots (Anti HIV 1 und Anti HIV 2) der Fa. DuPont angewendet. HCV wurde mit Hilfe von zwei verschiedenen ELISA-Verfahren nachgewiesen (HP 1 und HP 2).

Wie die Daten der Tab. 15 bis 17 deutlich machen, werden Anti HIV 1 und Anti HIV 2 sowie Anti HCV in Proben humanen Ursprungs mit dem erfindungsgemäßen Verfahren sicher und zuverlässig nachgewiesen.

Tab. 15

| Bestimmung von Anti HIV 1 mit dem erfindungsgemäßen Verfahren im Vergleich mit einem Anti HIV 1/2 Kombi- nationstest (Enzygnost[R] Anti HIV 1/2, HP 3) | | | |
|---|---|---|---|
| Stark reaktiv bedeutet Extinktionen von > 2,5 bei der photometrischen Auswertung; alle 76 Anti HIV 1-positiven Proben sind HCV-negativ | | | |
| Anzahl Proben | Herkunft der Probe | erfindungsgemäßes Verfahren | kommerzieller Anti HIV 1/2 Kombinationstest |
| n = 12 (Anti HIV 1 positiv) | Europa | n = 12 stark reaktiv | n = 12 stark reaktiv |
| n = 57 (Anti HIV 1 pos.) | West-Afrika | n = 57 stark reaktiv | n = 57 stark reaktiv |
| n = 7 (HIV 1/ HIV 2 Koinfektionen) | West-Afrika | n = 7 stark reaktiv | n = 7 stark reaktiv |
| n = 76 | | n = 76 positiv | n = 76 positiv |

Tab. 16

| Bestimmung von Anti HIV 2 mit den erfindungagemäßen Ver- fahren im Vergleich mit einem Anti HIV 1/2 Kombinations- test (Enzygnost[R] Anti HIV 1/2, HP 3) | | | |
|---|---|---|---|
| Stark reaktiv bedeutet Extinktionen von > 2,5 bei der photometrischen Auswertung; alle 28 Anti HIV 2- positiven Proben sind HCV negativ. | | | |
| Anzahl Proben | Herkunft der Proben | erfindungsgemäßes Verfahren | kommerzieller Anti HIV 1/2-Kombinationstest |
| n = 21 (Anti HIV 2 | West-Afrika | n = 21 stark reaktiv | n = 21 stark reaktiv positiv) |
| n = 7 (HIV 1/ HIV 2-Koinfektionen) | West-Afrika | n = 7 stark reaktiv | n = 7 stark reaktiv |
| n = 28 | | n = 28 positiv | n = 28 positiv |

Tab. 17

| Nachweis von Anti HCV mit dem erfindungsgemäßen ELISA- Verfahren | | | | |
|---|---|---|---|---|
| Als stark reaktiv wurden Proben bezeichnet, die Extinktionen von > 2,500 erzielten. Für die mittelstark reaktiven Proben werden sog. ratios angegeben, worunter der Quotient von Extinktion der Probe zu cut off eines gegebenen ELISAs verstanden wird. Werte unter 1,0 werden übereinkunftsgemäß als negativ bezeichnet. Werte über 1,0 als positiv, wobei die Reaktivität umso stärker ist, je größer ein ratio-Wert ausfällt; alle Anti HCV-positiven Proben sind HIV negativ | | | | |
| Anzahl Proben | Herkunft der Proben | erfindungsgemäßes Verfahren | kommerzielle Anti-HCV-ELISAs | |
| | | | HP 1 | HP 2 |
| n = 61 Anti HCV positiv | Europa | n = 57 stark reaktiv | n = 57 stark reaktiv | n = 57 stark reaktiv |
| | Europa | n = 4 | | |
| HC 90-346 | | > 9,0 ++ | 2,7 (+) | > 5,0 ++ |
| -351 | | 6,4 + | negativ | 3,9 + |
| -516 | | 6,8 + | negativ | 5,0 + |
| -570 | | 6,9 + | 1,9 (+) | > 5,0 + |
| n = 53 Anti HCV positiv | USA | n = 51 stark reaktiv | n = 51 stark reaktiv | n = 51 stark reaktiv |
| | USA | n = 2 | | |
| HC 90-511 | | 4,2 + | 1,2 (+) | 3,99 + |
| -566 | | 4,6 + | negativ | > 5,00 + |
| n = 114 | | n = 114 positiv | n = 111 positiv | n = 114 positiv |

Beispiel 11:

**Ermittlung der analytischen Sensitivität des erfindungsgemäßen ELISAs für Anti HIV und Anti HCV im Vergleich zum Stand der Technik**

Für die Beurteilung der analytischen Sensitivität des erfindungsgemäßen ELISAs wurden modellhaft Titrationen von positiven Proben hergestellt und in dem in Beispiel 9 beschriebenen ELISA getestet. Insgesamt wurden Verdünnungen von je drei Anti HIV 1- bzw. Anti HIV 2-positiven Human-Seren in Anti HIV- und Anti HCV- negativem Serum hergestellt und die Grenzempfindlichkeit als letzte im jeweiligen Testsystem noch reaktive Vorverdünnung definiert unter Verwendung des neuen Grenzwertes (Mittelwert der Negativ-Kontrollen plus 0,250 threshold).

Die Ergebnisse dieser Grenzempfindlichkeitsprüfung sind in Tab. 18 (Anti HIV 1), Tab. 19 (Anti HIV 2) und Tab. 20 (Anti HCV) als Extinktionswerte zusammengefaßt.

Tab. 18

| **Bestimmung der Grenzempfindlichkeit des erfindungsgemäßen ELISAs gegenüber Anti HIV $1_R$ im Vergleich zum Anti HIV 1/2 Kombinationstest (Enzygnost[R] Anti HIV 1/2).** | | | |
|---|---|---|---|
| Drei bestätigte Anti HIV 1-positive Proben wurden mit humanen Anti HIV 1- und Anti HCV-negativen Seren in Zweier-Stufen vorverdünnt und diese Verdünnungen gemäß Beispiel 3 bzw. laut Packungsbeilage des Herstellers des Vergleichstests getestet. Alle Angaben stellen Extinktionswerte dar ($E_{450}$). Alle Anti HIV-positiven Proben reagierten negativ in HP 2. | | | |
| Probe | Vorverdünnung | Extinktionen des erfindungsgemäßen ELISAs cut off=0,282 O.D. | Extinktionen des Anti HIV 1/2 Kombinationstests (HP3) cut off=0,268 O.D. |
| 8803/26 | unverdünnt | > 2,500 + | > 2,500 + |
| | 1 : 128 | > 2,500 + | > 2,500 + |
| Anti HIV 1-positiv | 256 | 2,334 + | 2,185 + |
| | 512 | 1,408 + | 1,412 + |
| | 1024 | 0,825 + | 0,837 + |
| | 2048 | 0,467 + | 0,457 + |
| | 4096 | 0,240 - | 0,216 - |
| | 8000 | 0,137 - | 0,135 - |
| 8611/144 | unverdünnt | > 2,500 + | > 2,500 + |
| | 1 : 128 | 2,162 + | 2,014 + |
| Anti HIV 1-positiv | 256 | 1,366 + | 1,028 + |
| | 512 | 0,806 + | 0,648 + |
| | 1024 | 0,496 + | 0,454 + |
| | 2048 | 0,280 - | 0,258 - |
| | 4096 | 0,153 - | 0,136 - |
| | 8000 | 0,088 - | 0,076 - |
| 8803/24 Anti HIV 1-positiv | 1 : 400 | 1,632 + | 1,585 + |
| 22281 Anti HCV-positiv | 1 : 256 | 2,219 + | 0,014 (negativ) |

Tab. 19

| **Bestimmung der Grenzempfindlichkeit des erfindungsgemäßen ELISAs gegenüber Anti HIV 2 im Vergleich zum Anti HIV 1/2 Kombinationstest (Enzygnost[R] Anti HIV 1/2).** | | | |
|---|---|---|---|
| Drei bestätigte Anti HIV 2-positive Proben wurden mit humanen Anti HIV- und Anti HCV-negativem Serum in Zweier-Stufen vorverdünnt und diese Verdünnungen gemäß Beispiel 3 bzw. laut Packungsbeilage des Herstellers des Vergleichstests getestet. Alle Angaben stellen Extinktionswerte dar ($E_{450}$). Alle Anti HIV-positiven Proben reagierten negativ in HP 2. | | | |
| Probe | Vorverdünnung | Extinktionen des erfindungsgemäßen ELISAs cut off=0,282 O.D. | Extinktionen des Anti HIV 1/2 Kombinationstests cut off=0,268 O.D. |
| 8804/103 | unverdünnt | > 2,500 + | > 2,500 + |
| | 1 : 128 | > 2,500 + | 2,500 + |

Tab. 19   (fortgesetzt)

| Bestimmung der Grenzempfindlichkeit des erfindungsgemäßen ELISAs gegenüber Anti HIV 2 im Vergleich zum Anti HIV 1/2 Kombinationstest (Enzygnost[R] Anti HIV 1/2). | | | |
|---|---|---|---|
| Probe | Vorverdünnung | Extinktionen des erfindungsgemäßen ELISAs cut off=0,282 O.D. | Extinktionen des Anti HIV 1/2 Kombinationstests cut off=0,268 O.D. |
| Anti HIV 2-positiv | 256 | 2,420 + | 2,045 + |
| | 512 | 1,488 + | 1,339 + |
| | 1024 | 0,737 + | 0,705 + |
| | 2048 | 0,365 + | 0.385 + |
| | 4000 | 0,196 - | 0,190 - |
| 8804/117 | unverdünnt | > 2,500 + | > 2,500 + |
| | 1 : 128 | > 2,500 + | > 2,500 + |
| Anti HIV 2-positiv | 256 | 1,860 + | 1,902 + |
| | 512 | 1,233 + | 1,100 + |
| | 1024 | 0,656 + | 0,667 + |
| | 2048 | 0.320 + | 0.302 + |
| | 4000 | 0,166 - | 0,172 - |
| 8804/116 Anti HIV 2-positiv | 1 : 400 | 1,045 + | 1,020 + |
| 22281 Anti HCV-positiv | 1 : 256 | 2,219 + | 0,014 (negativ) |

Tab. 20

| Bestimmung der Grenzempfindlichkeit des erfindungsgemäßen ELISAs gegenüber Anti HCV. | | | |
|---|---|---|---|
| Vier bestätigte Anti HCV-positive Proben wurden mit humanen Anti HIV- und Anti HCV-negativen Seren in Zweier-Stufen vorverdünnt und diese Verdünnungen gemäß Beispiel 3 getestet. Alle Angaben stellen Extinktionswerte dar ($E_{450}$ nm). | | | |
| Probe | Vorverdünnung | Extinktionen des erfindungsgemäßen ELISAs cut off=0,270 | kommerz. Anti HCV-ELISA (HP2) cut off=0,481 |
| HC 90-85 | unverdünnt | > 2,500 + | > 2,500 + |
| | 1 : 16 | > 2,500 + | > 2,500 + |
| Anti HCV-positiv | 1 : 32 | 2,299 + | > 2,500 + |
| | 1 : 64 | 1,858 + | > 2,500 + |
| | 1 : 128 | 1,515 + | > 2,500 + |
| | 1 : 256 | 1,045 + | 1,707 + |
| | 1 : 512 | 0,648 + | 0,871 + |
| | 1 : 1024 | 0.322 + | 0.492 + |
| | 1 : 2048 | 0,207 - | 0,255 - |
| HC 90-225 | unverdünnt | > 2,500 + | > 2,500 + |
| | 1 : 16 | > 2,500 + | > 2,500 + |

EP 0 484 787 B1

Tab. 20 (fortgesetzt)

| Bestimmung der Grenzempfindlichkeit des erfindungsgemäßen ELISAs gegenüber Anti HCV. | | | |
|---|---|---|---|
| Probe | Vorverdünnung | Extinktionen des erfindungsgemäßen ELISAs cut off=0,270 | kommerz. Anti HCV-ELISA (HP2) cut off=0,481 |
| Anti HCV-positiv | 1 : 32 | 2,214 + | > 2,500 + |
| | 1 : 64 | 1,816 + | > 2,500 + |
| | 1 : 128 | 1,488 + | 2,008 + |
| | 1 : 256 | 0,689 + | 1,120 + |
| | 1 : 512 | 0,580 + | 0.501 + |
| | 1 : 1024 | 0.286 + | 0,375 - |
| | 1 : 2048 | 0,148 - | 0,111 - |
| Anti HCV-positiv HC 90-270 | 1 : 256 | 0,999 + | 1,544 + |
| HC 90-354 | 1 : 128 | 0,971 + | 0,761 + |
| Anti HIV 1-positiv 8803/26 | unverdünnt | > 2,500 + | 0,110 - |
| 8811/144 | unverdünnt | > 2,500 + | 0,098 - |
| 8803/24 | unverdünnt | > 2,500 + | 0,068 -(negativ) |
| Anti HIV 2-positiv 8804/103 | unverdünnt | > 2,500 + | 0,075 - |
| 8804/117 | unverdünnt | > 2,500 + | 0,081 - |
| 8804/116 | unverdünnt | > 2,500 + | 0,099-(negativ) |

Analog wurde bei Anti HCV Verfahren, indem von vier Anti HCV-positiven Proben Verdünnungsreihen wie in Tab. 20 beschrieben hergestellt, getestet und analog zu Anti HIV ausgewertet und mit den Ergebnissen mit dem kommerziellen Anti HCV ELISA der 2. Generation (HP 2) verglichen wurden.

Zusätzlich wurden in den Einzeltests auch die Spezifität der Reaktivitäten von Einzelspenden geprüft, indem die Anti HIV 1- und Anti HIV 2-positiven Proben auch unverdünnt im Anti HCV-Test sowie umgekehrt die Anti HCV-positiven Proben in Anti HIV 1/2-Kombinationstests (Enzygnost[R] Anti HIV 1/2) untersucht wurden.

Die in Tab. 18 bis 20 zusammengefaßten Ergebnisse machen deutlich, daß die Grenzempfindlichkeit des erfindungsgemäßen ELISA sowohl bezüglich Anti HIV 1 als auch Anti HIV 2 der Grenzempfindlichkeit des Anti HIV 1/2- Kombinationstests entspricht. Auch entsprach die HCV-Grenzempfindlichkeit des erfindungsgemäßen ELISA der Grenzempfindlichkeit eines kommerziell erhältlichen Anti-HCV ELISAs (HP 2). Während jedoch für den Nachweis dieser drei Antikörper-Spezifitäten nach dem Stand der Technik insgesamt mindestens zwei Tests (Anti HIV 1/2-Kombinationstest sowie mindestens ein Anti HCV-Test) erforderlich sind, gelingt dieser Nachweis mit dem erfindungsgemäßen Verfahren ebenso zuverlässig und vergleichbar sensitiv mit nur einem Testansatz.

Ferner wird aus den Daten der Tab. 18 bis 20 ersichtlich, daß die starke Reaktivität der Anti HIV- und Anti HCV-positiven Proben im neuen ELISA besonders vorteilhaft ist, da die Anti HIV Proben negativ im spezifischen Anti HCV-Test und die Anti HCV-positiven Proben negativ im spezifischen Anti HIV-Test reagierten.

Beispiel 12

**Bestimmung von Anti HIV 1-positiven Proben aus frühen Infektionsstadien (Serokonversionen) mit dem erfindungsgemäßen ELISA.**

Getestet wurden insgesamt 3 Patienten, von denen im Verlauf sehr früher Phasen einer HIV 1-Infektion wiederholt sequentielle Blutentnahmen zu definierten Zeitpunkten gewonnen wurden. Diese Serumpanel sind kommerziell erhältlich (Boston Biomedica Inc., USA). In Tab. 21 wurden die Ergebnisse wiedergegeben, die mit dem erfindungsgemäßen ELISA im Vergleich zu einem Anti HIV 1/2 Kombinationstest erhalten wurden.

**Tab. 21:**

Alle Angaben stellen Extinktionswerte dar ($E_{450nm}$) wobei Werte oberhalb des jeweiligen Grenzwertes als positiv zu betrachten sind.

Tab. 21

| Proben-Code | erfindungsgem. ELISA | kommerz. Anti HIV 1/2 ELISA | kommerz. Anti HCV ELISA(HP2) |
|---|---|---|---|
| 1 A | 0,023 | 0,066 | negativ |
| 2 A | 0,020 | 0,055 | " |
| 3 A | 0,055 | 0,193 | " |
| 4 A | 1,163 + | > 2,500 + | " |
| 5 A | > 2,500 + | > 2,500 + | " |
| 6 A | > 2,500 + | > 2,500 + | " |
| 7 A | > 2,500 + | > 2,500 + | " |
| 8 A | > 2,500 + | > 2,500 + | " |
| 9 A | > 2,500 + | > 2,500 + | " |
| 1 C | 0,014 | 0,022 | negativ |
| 2 C | 0,015 | 0,054 | " |
| 3 C | 0,054 | 0,145 | " |
| 4 C | 0,500 + | 1,138 + | " |
| 5 C | 0,630 + | 1,707 + | " |
| 6 C | 0,889 + | > 2,500 + | " |
| 7 C | 0,942 + | " | " |
| 8 C | 1,010 + | " | " |
| 9 C | 1,081 + | " | " |
| 10 C | 1,564 + | " | " |
| 11 C | > 2,500 + | " | " |
| 12 C | " | " | " |
| 13 C | " | " | " |
| 14 C | " | " | " |
| 15 C | " | " | " |
| 16 C | " | " | " |
| 17 C | " | " | " |
| 18 C | " | " | " |
| G BBI | | | |
| 80 | 0,012 | 0,056 | negativ |
| 81 | 0,013 | 0,053 | " |
| 82 | 0,018 | 0,059 | " |
| 83 | 0,478 + | 1,112 + | " |
| 84 | 0,733 + | > 2,500 + | " |
| 85 | 0,612 + | > 2,500 + | " |
| 86 | 0,763 + | > 2,500 + | " |
| 87 | 1,296 + | > 2,500 + | " |
| 88 | 1,309 + | > 2,500 + | " |
| 89 | 1,544 + | > 2,500 + | " |
| Grenzwert | 0,281 | 0,285 | 0,481 |

Die Ergebnisse der Tab. 21 machen deutlich, daß der erfindungsgemäße ELISA niedrig-titrige Anti HIV 1-positive Proben ebenso zuverlässig nachweist, wie der Anti HIV 1/2 Kombinationstest (Enzygnost[R] Anti HIV 1/2). Ebenso wird aus dem Vergleich mit den Daten der Tab. 21 ersichtlich, daß auch der früheste Zeitpunkt, an dem mit dem neuen Peptid-ELISA der erste Nachweis von HIV 1-spezifischen Antikörpern gelingt, mindestens so früh möglich ist, wie mit spezifischen singulären Anti HIV 1-Tests als Stand der Technik.

Beispiel 13

**Bestimmung von niedrig-titrigen Anti HIV 1-positiven Proben mit dem erfindungsgemäßen ELISA**

In Tab. 22 sind die Ergebnisse zusammengefaßt, die bei der Testung eines kommerziell erhältlichen "Anti HIV low titre - Panel" mit dem neuen HIV/HCV-Peptid ELISA erhalten wurden im Vergleich zu einem Anti HIV 1/2-Kombinationstest (Enzygnost[R] Anti HIV 1/2).

Das Proben-Panel der Fa. Boston Biomedica, Inc., USA umfaßt insgesamt 15 Proben, die mit Hilfe von Anti HIV 1-Bestimmungsmethoden als niedrig-titrig Anti HIV 1-positiv klassifiziert wurden.

**Tab. 22**

**Ergebnisse der Testung des "low titre Anti HIV 1-Panel" der Fa. Boston Biomedica Inc., USA**

Alle Angaben stellen Extinktionswerte bei einer Wellenlänge von 450 nm dar. Werte über den angegebenen Grenzwerten sind positiv.

Tab. 22

| ID No. | erfindungsgemäßer ELISA | kommerzieller Anti HIV 1/2 ELISA HCV | kommerzieller Anti HCV ELISA |
|---|---|---|---|
| BO 1-01 | > 2,500 | 1,872 | > 2,500 + |
| 02 | 1,979 | 1,575 | 2.023 + |
| 03 | 2,056 | 2,280 | negativ |
| 04 | 1,751 | 1,960 | " |
| 05 | 1,781 | 2,044 | " |
| 06 | 1,428 | 1,728 | " |
| 07 | 1,183 | 1,564 | " |
| 08 | 0,641 | 1,152 | " |
| 09 | 0,984 | 1,499 | " |
| 10 | 0,682 | 0,940 | " |
| 11 | 1,302 | 1,650 | " |
| 12 | 2,032 | 0,540 | 1,833 + |
| 13 | 1,017 | 1,854 | negativ |
| 14 | 1,079 | 1,484 | " |
| 15 | > 2,500 | 2,090 | > 2,500 + |
| cut off | 0,279 | 0,265 | 0,479 |

Wie die Ergebnisse der Tab. 22 erkennen lassen, werden im Vergleich zu dem Anti HIV 1/2-Kombinationstest (Enzygnost[R] Anti HIV 1/2) mit dem erfindungsgemäßen HIV/HCV-Peptid ELISA alle Proben vergleichbar stark reaktiv ermittelt.

Interessanterweise werden vier Proben des Panels mit dem neuen Peptid-ELISA sogar stärker reaktiv ermittelt als mit dem Anti HIV 1/2-Kombinationstest (Enzygnost[R] Anti HIV 1/2) (No. 1, 2, 12 und 15). Zusätzliche Prüfungen dieser Proben ergaben gleichzeitig vorliegende HCV-Antikörper, was die Zuverlässigkeit eines nicht-differenzierenden Nachweis von Anti-HIV 1/-HIV 2 und -HCV nach dem erfindungsgemäßen Verfahren erneut bestätigt.

**Beispiel 14**

**Bestimmung von niedrig-titrigen Anti HCV-positiven Proben mit dem erfindungsgemäßen ELISA.**

Tab. 23 enthält die Ergebnisse, die mit dem neuen HIV 1-/ HIV 2- und HCV-Peptid ELISA und dem low titre Anti HCV-Panel der Firma Boston Biomedica Inc., USA, erhalten wurden. In dieser Tab. 23 sind auch Daten enthalten, die am gleichen Panel mit einem modernen Anti HCV ELISA (HP 2) erhoben wurden.

Die Angaben zu dem erfindungsgemäßen und kommerziellen ELISA stellen sogenannte Endpunkt-Titer dar, mit denen die höchste Vorverdünnung eines Serums in Anti HCV-negativem Serum definiert wird, die in einem gegebenen Test reproduzierbar positiv ermittelt wurde.

Tab. 23:

| BBI low titre Anti HCV PANEL PHV 101 | | |
|---|---|---|
| MEMBER I.D. NUMBER | erfindungsgemäßer ELISA | kommerzieller Anti HCV-Test (HP 2) |
| PHV101-01 | 1 : 16 | 1 : 2 |
| PHV101-02 | negativ | negativ |
| PHV101-03 | 1 : 4 | 1 : 1 |
| PHV101-04 | 1 : 512 | 1 : 64 |
| PHV101-05 | 1 : 2 | 1 : 1 |
| PHV101-06 | 1 : 128 | 1 : 32 |
| PHV101-07 | 1 : 128 | 1 : 128 |
| PHV101-08 | 1 : 64 | 1 : 16 |
| PHV101-09 | 1 : 64 | 1 : 16 |
| PHV101-10 | 1 : 256 | 1 : 128 |
| PHV101-11 | 1 : 32 | 1 : 16 |
| PHV101-12 | 1 : 512 | 1 : 128 |
| PHV101-13 | 1 : 16 | 1 : 64 |
| PHV101-14 | 1 : 64 | 1 : 32 |
| PHV101-15 | 1 : 128 | 1 : 64 |

Die Ergebnisse der Tab. 23 machen deutlich, daß mit dem erfindungsgemäßen ELISA alle 14 positiven Proben zuverlässig eindeutig positiv ermittelt wurden. Dabei fällt auf, daß die Signalstärke sehr hoch ist, was auf eine große Reaktivität zurückzuführen ist.

Diese hohe Reaktivität spiegelt sich auch wieder in den ermittelten Grenzempfindlichkeiten der Tab. 23. Diese Nachweisgrenzen wurden definiert, indem die nativen Humanproben in Zweier-Schritten in Anti HCV-negativem Serum vorverdünnt und dann gemäß Beispiel 4 geprüft wurden, wobei die letzte noch reaktiv ermittelte Vorverdünnungsstufe den sogenannten Endtiter darstellt.

Auf dieser Basis lassen die Daten der Tab. 23 erkennen, daß der neue HIV 1-/HIV 2-/HCV-Peptid ELISA in dem Panel PHV 101 bezüglich Anti HCV sogar bessere Nachweisgrenzen aufweist als der Stand der Technik.

Beispiel 15

**Bestimmung der Quote unspezifischer Reaktionen des neuen Peptid-ELISA in einem Kollektiv gesunder Blutspender**

Für die Ermittlung der Häufigkeit von unspezifischen Reaktionen, die im ELISA zu falsch positiven Ergebnissen führen, wurden insgesamt n = 512 gesunde Blutspender herangezogen. Von diesen Spendern wurde gleichzeitig Serum und Plasma gewonnen, um auch einen möglichen Störeinfluß des Gerinnungssystems auf das Verfahren untersuchen zu können.

Proben, die in einem dieser drei Tests (erfindungsgemäßer ELISA, Anti HCV ELISA HP 2 und Anti HIV 1/2 HP 3) reaktiv waren (sog. Initial-Testung) wurden im gleichen Test wiederholt (sog. Retesting) und bei reproduzierbarer Reaktivität im Anti HIV 1 Western blot und Anti HCV-ELISA (HP 1) als Vergleichsverfahren untersucht.

Keine der in diesem Screening als reaktiv in einem der drei ELISAs aufgefallenen Proben konnte mit Hilfe dieser Bestätigungsverfahren als Anti HIV 1- oder als Anti HCV-positiv bestätigt werden. So wurden alle Proben, die im Screening aussortiert wurden, folgerichtig als falsch-positiv in den jeweiligen Verfahren klassifiziert.

Tab. 24:

Screening von n = 512 gesunden Blutspendern, von denen gleichzeitig 512 Sera und 512 sog. "paired plasma" gewonnen wurden. Angegeben sind die Befunde nach Initial-Testung; zu Retest-Ergebnissen siehe Tab. 25.

**Tab. 24**

| Proben-Code | erfindumgsgem. Verfahren Anzahl reaktiver Seren/ Plasmen | ratios Seren/ Plasmen | Anti HIV 1/2 Anzahl reaktiver Seren/ Plasmen | ratios Seren/ Plasmen | Anti HCV (HP 2) Anzahl reaktiver Seren/ Plasmen | ratios Seren/ Plasmen |
|---|---|---|---|---|---|---|
| | 3 / 2 | | 0 / 0 | | 0 / 0 | |
| 9104-PE-1 | | 2,1 / 1,3 | | neg. | | neg. |
| 2 | | 1,0 / 1,2 | | neg. | | neg. |
| 3 | | 1,1 / 0,9 - | | neg. | | neg. |
| | 0 / 0 | | 1 / 1 | | | |
| 9104-HIV-1 | | neg. | | 1,8 / 1,6 | | neg. |
| | 0 / 0 | | 0 / 0 | | 5 / 4 | |
| 9104-HCV-1 | | neg. | | neg. | | >4,0 / 3,5 |
| 2 | | neg. | | neg. | | 2,5 / 3,0 |
| 3 | | neg. | | neg. | | 1,4 / 1,2 |
| 4 | | neg. | | neg. | | 1,3 / 1,5 |
| 5 | | neg. | | neg. | | 0,8 - / 1,1 |

EP 0 484 787 B1

**Tab. 25** Ergebnisse der Spezifität nach wiederholter Prüfung der 9 reaktiven Seren aus Tab. 24

| n = 512 Seren und n = 512 paired Plasmen von insgesamt 512 gesunden Blutspendern | neuer Peptid-ELISA | Anti HIV 1/2 (HP 3) | Anti HCV (HP 2) |
|---|---|---|---|
| | Serum / Plasma | Serum / Plasma | Serum / Plasma |
| Anzahl falsch positiver Resultate Initialtestung | 3 / 2 | 1 / 1 | 5 / 4 |
| initiale Spezifität (%) | 99,41 / 99,60 | 99,80 / 99,80 | 99,02 / 99,22 |
| Anzahl falsch positiver Resultate nach Wiederholung | 2 / 2 | 1 / 1 | 4 / 4 |
| retest-Spezifität (%) | 99,60 / 99,60 | 99,80 / 99,80 | 99,22 / 99,22 |

EP 0 484 787 B1

Die Ergebnisse der ersten Versuchsreihe (Initial-Ergebnisse) sind in Tab. 24 zusammengefaßt. Während nach dem erfindungsgemäßen Verfahren drei Seren (und zwei korrespondierende Plasmen) reaktiv waren, wurden mit einem Anti HIV 1/2-ELISA 1 Serum (und ein korrespondierendes Plasma) und dem Anti HCV-ELISA fünf Seren (und vier korrespondierende Plasmen) reaktiv ermittelt. Die in dem jeweiligen ELISA reaktiven Spender waren nicht identisch.

Nach wiederholter Versuchsdurchführung dieser neun reaktiven Seren ergab sich das in Tab. 25 dargestellte Bild von zwei Seren (zwei paired Plasmen), die im erfindungsgemäßen Peptid-ELISA "retest-reaktiv" waren, ein Serum (ein "paired" Plasma), das reaktiv in dem Anti HIV 1/2-ELISA war sowie vier Seren (vier paired Plasmen), die im Anti HCV-ELISA reaktiv waren.

Unter Zugrundelegung, daß sich keine dieser Proben als Anti HIV- und/oder Anti HCV-positiv bestätigen ließ, wurden die in Tab. 25 dargestellten Spezifitätsdaten für jeden der untersuchten ELISAs sowie für Seren und Plasmen ermittelt, indem der prozentuale Anteil der richtig negativ ermittelten Proben (insgesamt je 512 Seren und Plasmen) errechnet wurde.

Basierend auf den Daten der Tab. 25 hätten gemäß dem Stand der Technik - obligatorische Prüfung jeder Spende auf Anti HIV und Anti HCV - insgesamt fünf Spender ausgeschlossen werden müssen:
Ein Spender wiederholbar falsch positiv im Anti HIV 1/2-Test und vier Spender wiederholbar falsch positiv im Anti HCV-Test. Demgegenüber ist die Zahl der falsch reaktiv ermittelbaren Spender im erfindungsgemäßen ELISA nur zwei.

Zusammenfassend läßt sich sagen, daß der neue Peptid-ELISA zum gleichzeitigen nicht-differenzierenden Nachweis von Anti HIV 1, Anti HIV 2 und Anti HCV bezüglich den z. Z. gültigen Kriterien der Sensitivität den jeweils optimalen Leistungsmerkmalen der zwei Einzeltests Anti HIV 1/2 einerseits und Anti HCV andererseits mindestens entspricht: die Daten der Testung von Panels, d. h. nativen Proben enthaltend Anti HIV 1 und/oder Anti HIV 2 und/oder Anti HCV verweisen ebenso auf vergleichbare Effizienz wie die Prüfung der Grenzempfindlichkeiten und Zeitpunkt der frühesten Erkennung niedrig-titriger Anti HIV 1- oder Anti HIV 2- oder Anti HCV-Proben. Während der Nachweis dieser drei Antikörper-Spezifitäten gemäß dem Stand der Technik in dieser Weise nur mit drei verschiedenen oder im Hinblick auf die Anti HIV 1/2 Kombinationstests mit zwei verschiedenen Test möglich ist, bietet der erfindungsgemäße ELISA den Vorteil, bei gleicher Effizienz nur einen Test durchführen zu müssen. Gerade für die Blutbanken, die zu einer Testung auf Anti HIV 1, Anti HIV 2 und Anti HCV verpflichtet sind, bedeutet der neue Peptid-ELISA eine erhebliche Reduktion von Aufwand und Kosten bei mindestens gleicher Zuverlässigkeit und Sicherheit bei der Bestimmung von Anti HIV 1, Anti HIV 2 und Anti HCV.

Völlig überraschend wurde außerdem bei der Ermittlung der Störanfälligkeit dieses neuen Tests gefunden, daß aufgrund der sehr guten Spezifität, d. h. nur geringe Zahl falsch positiver Befunde, weniger Nachtestungen sowie weniger Bestätigungstests erforderlich sind und auch weniger Spenden ausgeschlossen werden müssen, als bei dem herkömmlichen Screening-Vorgehen der Verwendung eines Anti HIV 1/2-Tests und getrennt davon eines Anti HCV-Tests. Damit bietet das erfindungsgemäße Verfahren wirtschaftliche und fachliche Vorteile, die auch andere Konstellationen von HIV- und HCV-Peptiden der Formeln IV bis XII bzw. XIII bis XVII bieten.

Auch für andere Fragestellungen, in denen maximal optimierte Grenzempfindlichkeit die wichtigste Rolle spielt, ist das erfindungsgemäße Verfahren bestens geeignet. So kann unter anderen Testbedingungen oder z. B. rechnerisch in den Beispielen 10 bis 14 gezeigt werden, daß es durchaus möglich ist, Sensitivitätsmerkmale des erfindungsgemäßen Verfahrens zu erzeugen, die signifikant besser sind als die des Stands der Technik, wenn etwas ungünstigere Spezifitätsdaten in Kauf genommen werden, die aber dann immer noch dem Stand der Technik entsprechen.

Würde man beispielsweise den Grenzwert (Beispiel 9) auf 0,1 Extinktion erniedrigen, wären alle Grenzempfindlichkeiten für Anti HIV 1, Anti HIV 2 und Anti HCV um den Faktor von mindestens 2 erheblich verbessert. Für die Quote der unspezifisch, d. h. falsch positiv reagierenden Proben im Screening der gesunden Blutspender (Beispiel 15) würden insgesamt fünf Spender resultieren, die wiederholbar reaktiv ermittelt wurden, was dem Ergebnis aus beiden Einzeltests absolut entspricht, wobei allerdings die Empfindlichkeit des Antikörper-Nachweises wesentlich verbessert wurde.

**Patentansprüche**

**1.** Peptide, die spezifisch mit Antikörpern gegen HCV reagieren, **dadurch gekennzeichnet, daß** ihre Aminosäuresequenzen mindestens einer der folgenden Sequenzen entsprechen:

4073:

128                                                                 160

PDREVLYREFDEMEECSQHLPYIEQGMMLAEQF

4071:

144                          160

SQHLPYIEQGMMLAEQF

4081:

                                    161                     175

                                    KQKALGLLQTASRQA

4082:

144                                                         175

SQHLPYIEQGMMLAEQFKQKALGLLQTASRQA

4090:

135                                          164

REFDEMEECSQHLPYIEQGMMLAEQFKQKA


4055:

129                143

DREVLYREFDEMEEC


4053:

145                159

QHLPYIEQGMMLAEQ


4052:

153                167

GMMLAEQFKQKALGL


4060:

121                139

SGKPAIIPDREVLYREFDE

4083:

121                                                             175

SGKPAIIPDREVLYREFDEMEECSQHLPYIEQGMMLAEQFKQKALGLLQTASRQA


4056:

121                135

SGKPAIIPDREVLYR


2.   Peptide, die spezifisch mit Antikörpern gegen HCV reagieren, **dadurch gekennzeichnet, daß** sie mindestens eine der folgenden Aminosäure-sequenzen enthalten

$$AS_{a1}\text{-DREVLYR-}BS_{b1}$$

$$AS_{a2}\text{-QHLPYIE-}BS_{b2}$$

$$AS_{a3}\text{-KQKALGL-}BS_{b3}$$

wobei AS und BS eine beliebige Aminosäure bedeuten und a1 bis a3 und b1 bis b3 jeweils unabhängig voneinander ganze Zahlen größer oder gleich Null sind und $AS_{a1}$, $BS_{b1}$, $AS_{a2}$, $BS_{b2}$, $AS_{a3}$ und $BS_{b3}$ nicht mit der entsprechenden Sequenz des C-100-3 Genomabschnitts von HCV übereinstimmen.

**Claims**

1. A peptide which reacts specifically with antibodies against HCV and whose amino-acid sequence corresponds to at least one of the following sequences:

<u>4073:</u>

128                                  160

PDREVLYREFDEMEECSQHLPYIEQGMMLAEQF

<u>4071:</u>

144               160

SQHLPYIEQGMMLAEQF

<u>4081:</u>

                          161              175

KQKALGLLQTASRQA

<u>4082:</u>

144                                          175

SQHLPYIEQGMMLAEQFKQKALGLLQTASRQA

<u>4090:</u>

135 164

REFDEMEECSQHLPYIEQGMMLAEQFKQKA

<u>4055:</u>

129 143

DREVLYREFDEMEEC

<u>4053:</u>

145 159

QHLPYIEQGMMLAEQ

<u>4052:</u>

153 167

GMMLAEQFKQKALGL

<u>4060:</u>

121 139

SGKPAIIPDREVLYREFDE

<u>4083:</u>

121 175

SGKPAIIPDREVLYREFDEMEECSQHLPYIEQGMMLAEQFKQKALGLLQTASRQA

<u>4056:</u>

121 135

SGKPAIIPDREVLYR

2. A peptide which reacts specifically with antibodies against HCV and which contains at least one of the following amino-acid sequences

$$AA_{a1}\text{-DREVLYR-}BA_{b1}$$

$$AA_{a2}\text{-QHLPYIE-}BA_{b2}$$

$$AA_{a3}\text{-KQKALGL-}BA_{b3}$$

where AA and BA are any desired amino acid, and a1 to a3 and b1 to b3 are each, independently of one another, integers greater than or equal to zero and $AA_{a1}$, $BA_{b1}$, $AA_{a2}$, $BA_{b2}$, $AA_{a3}$ and $BA_{b3}$ do not coincide with the corresponding sequence of the C-100-3 genome section of HCV.

**Revendications**

1. Peptides qui réagissent spécifiquement avec des anticorps dirigés contre HCV (virus de l'hépatite C), **caractérisés en ce que** leur séquence d'aminoacides correspond au moins à l'une des séquences suivantes :

<u>4073:</u>

128                                                    160

PDREVLYREFDEMEECSQHLPYIEQGMMLAEQF

<u>4071:</u>

144                        160

SQHLPYIEQGMMLAEQF

<u>4081:</u>

161                    175

KQKALGLLQTASRQA

<u>4082:</u>

144                                        175

SQHLPYIEQGMMLAEQFKQKALGLLQTASRQA

<u>4090:</u>

135                                        164

REFDEMEECSQHLPYIEQGMMLAEQFKQKA

<u>4055:</u>

129          143

DREVLYREFDEMEEC

<u>4053:</u>

145              159

QHLPYIEQGMMLAEQ

<u>4052:</u>

153          167

GMMLAEQFKQKALGL

<u>4060:</u>

121             139

SGKPAIIPDREVLYREFDE

<u>4083:</u>

121                           175

SGKPAIIPDREVLYREFDEMEECSQHLPYIEQGMMLAEQFKQKALGLLQTASRQA

<u>4056:</u>

121        135

SGKPAIIPDREVLYR

**2.** Peptides qui réagissent spécifiquement avec des anticorps dirigés contre HCV, **caractérisés en ce qu'**ils contiennent au moins l'une des séquences d'aminoacides suivantes

$$AS_{a1}\text{-DREVLYR-}BS_{b1} \qquad (S)$$

$$AS_{a2}\text{-QHLPYIE-}BS_{b2} \qquad (F1)$$

$$AS_{a3}\text{-KQKALGL-}BS_{b3} \qquad (F2)$$

AS et BS signifiant un aminoacide quelconque et a1 à a3 et b1 à b3 étant, indépendamment les uns des autres, des nombres entiers supérieurs ou égaux à zéro, et $AS_{a1}$, $BS_{b1}$, $AS_{a2}$, $BS_{b2}$, $AS_{a3}$ et $BS_{b3}$ ne coïncidant pas avec la séquence correspondante du segment de génome C-100-3 de HCV.

# Fig. 1

## Primärsequenz der Aminosäuren der ORF-Region von C-100-3

AS 1

```
D A H F L S Q T K Q S G E N L P Y L V A          20
Y Q A T V C A R A Q A P P P S W D Q M W          40
K C L I R L K P T L H G P T P L L Y R L          60
G A V Q N E I T L T H P V T K Y I M T C          80
M S A D L E V V T S T W V L V G G V L A         100


A L A A Y C L S T G C V V I V G R V V L         120
S G K P A I I P D R E V L Y R E F D E M         140
E E C S Q H L P Y I E Q G M M L A E Q F         160
K Q K A L G L L Q T A S R Q A E V I A P         180
A V Q T N W Q K L E T F W A K H M W N F         200


I S G I Q Y L A G L S T L P G N P A I A         220
S L M A F T A A V T S P L T T S Q T L L         240
F N I L G G W V A A Q L A A P G A A T A         260
F V G A G L A G A A I G S V G L G K V L         280
I D I L A G Y G A G V A G A L V A F K I         300


M S G E V P S T E D L V N L L P A I L S         320
P G A L V V G V V C A A I L R R H V G P         340
G E G A V Q W M N R L I A F A S R G N H         360
V S P
```

AS 363

## Fig. 2: Vergleich der erfindungsgemäßen Sequenzen der Formel I und II mit bekannten Peptiden aus der ORF-Region von C-100-3

Amino-Terminal     126 [========] 167     Carboxy-Terminal     5-1-1

C-100-3

AS 1 [=====================================] AS 363

126 [========] 167     sp 42

121 [===========] 237     sp 117

116 [============] 182     sp 67

298 [==========] 362     sp 65

Erfindungsgemäße Sequenzen

121 [=========] 175

337 [====] 363

Formel I         Formel II

## _Fig. 3:_ Vergleich der erfindungsgemäßen Sequenzen des HCV-core-Proteins mit bekannten Peptiden

Aminosäure 1 bis 35:
(Gegenstand der
vorliegenden Erfindung)

Aminosäure 39 bis 74:
(OKAMOTO et al.)

1    10    20    30    40    50    60    70

MSTNPKPQRKTKRNTNRRPQDVKFPGGGQIVGGVYLLPRRGPRLGVRATRKTSERSQPRGRRQPIPKARRPEGRT

Aminosäure
47 - 75 = sp 12

Aminosäure
1 - 30 = sp 10

Aminosäure
24 - 53 = sp 11

80    90    100    110    120

WAQPGYPWPLYGNEGCGWAGWLLSPRGSRPSWGPTDPRRRSRNLG

EP 0 484 787 B1